# EUROPEAN PATENT SPECIFICATION

(11) **EP 3 870 696 B1**
(45) Date of publication and mention of the grant of the patent: **26.03.2025**
(21) Application number: 19874787.5
(22) Date of filing: 24.10.2019
(51) Int. Cl.: C12N 9/22, C12P 19/34, C12Q 1/6853, C40B 50/06, C12Q 1/6806

(54) **METHODS AND KITS FOR DEPLETION AND ENRICHMENT OF NUCLEIC ACID SEQUENCES**
VERFAHREN UND KITS ZUR ABREICHERUNG UND ANREICHERUNG VON NUKLEINSÄURESEQUENZEN
PROCÉDÉS ET KITS POUR APPAUVRIR ET ENRICHIR DES MOLÉCULES D'ACIDE NUCLÉIQUE

(30) Priority: 24.10.2018 US 201862750169 P
(43) Date of publication of application: 01.09.2021
(73) Proprietor: University of Washington, Seattle, WA 98105 (US)
(72) Inventor: ROSENBERG, Alexander, B., Seattle, WA 98105 (US); ROCO, Charles, Seattle, WA 98105 (US); SEELIG, Georg, Seattle, WA 98105 (US)
(74) Representative: Titmus, Craig Edward
(86) International application number: PCT/US2019/057939
(87) International publication number: WO 2020/086896

(56) References cited:
- WO-A1-02/16647
- WO-A1-2013/112923
- WO-A1-2014/143228
- WO-A2-2007/030759
- WO-A2-2013/191775
- WO-A2-2013/191775
- US-A1- 2005 272 070
- US-A1- 2010 184 068

## Description

### BACKGROUND

Complex biological samples, such as tissues, cells, cell lysates, serum, and the like, present challenges to determining sequences and concentrations of nucleic acid molecules bearing particular target sequences. Likewise, determining sequences and concentrations of nucleic acids from a set of barcoded molecules, such as single-cell RNA sequencing libraries present similar challenges.

Conventionally, sequencing methods, such as Sanger sequencing or Next-Generation Sequencing (NGS) methods are used to sequence nucleic acids in such complex samples and sequence libraries, where large numbers of excess sequences are generated in addition to those based upon a target sequence of interest. Additionally, where NGS methods are used, relatively high numbers of sequencing reads are used to achieve a desired sequencing depth.

Selectively enriching for target nucleic acid sequences or depleting non-target nucleic acid sequences in complex samples would simplify interpreting sequence data and reduce a number of reads needed to achieve a particular sequencing depth.

Accordingly, there is presently a need in the art to selectively remove some or all nucleic acid molecules that are not of interest or selectively increase a proportion of nucleic acid molecules that are of interest in complex mixtures, such as in preparation for sequencing. The present disclosure seeks to fulfill these needs and provides further related advantages.

WO 2013/191775 mentions compositions and methods for negative selection of non-desired nucleic acid sequences. WO 02/16647 mentions methods and compositions for identifying nucleic acid molecules using nucleolytic activities and hybridization. US 2010/184068 mentions a method of measuring reverse-transcribed single stranded DNA, method of measuring reverse transcriptase activity and kit for the same.

### SUMMARY

The invention is defined according to the claims.

The present disclosure provides methods and kits for enriching target nucleic acid molecules.

Further disclosed are methods and kits for depleting nucleic acid molecules that are not of interest.

The invention provides a method for enriching a target nucleic acid sequence , comprising (a) introducing to a sample solution, comprising a plurality of sample nucleic acid molecules each comprising a universal adaptor nucleic acid sequence, a capture primer nucleic acid molecule complementary to or partially complementary to a target nucleic acid sequence of one or more sample nucleic acid molecules of the plurality of sample nucleic acid molecules; (b) enzymatically extending the capture primer nucleic acid molecule annealed to the target nucleic acid sequence of the one or more sample nucleic acid molecules; and (c) enzymatically degrading single-stranded sample nucleic acid molecules, to provide an enriched sample solution having a higher proportion of sample nucleic acid molecules comprising the target nucleic acid sequence than the sample solution.

Also disclosed is a method for depleting a target nucleic acid sequence. The method may comprise introducing to a sample solution, comprising a plurality of sample nucleic acid molecules each comprising a universal adaptor nucleic acid sequence comprising ribonucleotides, a capture primer nucleic acid molecule complementary or partially complementary to a target nucleic acid sequence of one or more sample nucleic acid molecules of the plurality of sample nucleic acid molecules; enzymatically extending the capture primer nucleic acid molecule annealed to the target nucleic acid sequence of the one or more sample nucleic acid molecules; and enzymatically cleaving double-stranded ribonucleic acid molecules of the sample nucleic acid molecules, to provide a depleted sample solution having a lower proportion of sample nucleic acid molecules comprising the target nucleic acid sequence than the sample solution.

Further disclosed is a kit for enriching a target nucleic acid sequence. The kit may comprise a capture primer nucleic acid molecule complementary to or partially complementary to a target sequence; and a degradation enzyme configured to degrade a single-stranded nucleic acid molecule.

A kit for depleting a target nucleic acid sequence is also disclosed. The kit may comprise a capture primer nucleic acid molecule complementary to or partially complementary to a target sequence; and a degradation enzyme configured to degrade a double-stranded nucleic acid molecule.

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

### DESCRIPTION OF THE DRAWINGS

The foregoing aspects and many of the attendant advantages of this disclosure will become more readily appreciated as the same become better understood by reference to the following detailed description, when taken in conjunction with the accompanying drawings, wherein:
FIGURE 1A schematically illustrates a sample solution including sample nucleic acid molecules to enrich and nucleic acid molecules to deplete, in accordance with an embodiment of the disclosure.
FIGURE 1B schematically illustrates the sample solution of FIGURE 1A further including capture primer nucleic acid molecules, in accordance with an embodiment of the disclosure.
FIGURE 1C schematically illustrates the sample solution of FIGURE 1B after melting the sample nucleic acid molecules to enrich and to deplete, in accordance with an embodiment of the disclosure.
FIGURE 1D schematically illustrates the sample solution of FIGURE 1C after annealing a capture primer nucleic acid molecule to a target sequence of a nucleic acid molecule to be enriched, in accordance with an embodiment of the disclosure.
FIGURE 1E schematically illustrates the sample solution of FIGURE 1D after enzymatically extending the capture primer nucleic acid molecule annealed to the target sequence, in accordance with an embodiment of the disclosure.
FIGURE 1F schematically illustrates the sample solution of FIGURE 1E after enzymatically degrading single-stranded sample nucleic acid molecules in the sample solution, in accordance with an embodiment of the disclosure.
FIGURE 1G schematically illustrates melting the nucleic acid molecules of the sample solution of FIGURE 1F, in accordance with an embodiment of the disclosure.
FIGURE 1H schematically illustrates the sample solution of FIGURE 1G after removing the capture primer nucleic acid molecules, in accordance with an embodiment of the disclosure.
FIGURE 1I schematically illustrates the sample solution of FIGURE 1H further including polymerase chain reaction (PCR) primers complementary to a universal adaptor sequence of the sample nucleic acid molecules in the sample solution, in accordance with an embodiment of the disclosure.
FIGURE 1J schematically illustrates the sample solution of FIGURE 1I after PCR amplification of certain sample nucleic acid molecules of the sample solution, in accordance with an embodiment of the disclosure.
FIGURE 2A schematically illustrates a sample solution including sample nucleic acid molecules to enrich and nucleic acid molecules to deplete, in accordance with the disclosure.
FIGURE 2B schematically illustrates the sample solution of FIGURE 2A further including capture primer nucleic acid molecules, in accordance with the disclosure.
FIGURE 2C schematically illustrates the sample solution of FIGURE 2B after melting the sample nucleic acid molecules to enrich and to deplete, in accordance with the disclosure.
FIGURE 2D schematically illustrates the sample solution of FIGURE 2C after annealing a capture primer nucleic acid molecule to a target sequence of a nucleic acid molecule to be depleted, in accordance with the disclosure.
FIGURE 2E schematically illustrates the sample solution of FIGURE 2D after enzymatically extending the capture primer nucleic acid molecule annealed to the target sequence, in accordance with the disclosure.
FIGURE 2F schematically illustrates the sample solution of FIGURE 2E after enzymatically degrading double-stranded sample nucleic acid molecules in the sample solution, in accordance with the disclosure.
FIGURE 2G schematically illustrates melting the sample nucleic acid molecules of the sample solution of FIGURE 2F, in accordance with the disclosure.
FIGURE 2H schematically illustrates the sample solution of FIGURE 2G after removing the capture primer nucleic acid molecules, in accordance with the disclosure.
FIGURE 2I schematically illustrates the sample solution of FIGURE 2H further including PCR primers complementary to a universal adaptor sequence of the sample nucleic acid molecules in the sample solution, in accordance with the disclosure.
FIGURE 2J schematically illustrates the sample solution of FIGURE 2I after PCR amplification of the sample nucleic acid molecules of the sample solution, in accordance with the disclosure.
FIGURE 3A schematically illustrates a sample solution including sample nucleic acid molecules to enrich and nucleic acid molecules to deplete, in accordance with the disclosure.
FIGURE 3B schematically illustrates the sample solution of FIGURE 3A further including capture primer nucleic acid molecules including blocked capture primer nucleic acid molecules, in accordance with the disclosure.
FIGURE 3C schematically illustrates the sample solution of FIGURE 3B after melting the sample nucleic acid molecules to enrich and to deplete, in accordance with the disclosure.
FIGURE 3D schematically illustrates the sample solution of FIGURE 3C after annealing a capture primer nucleic acid molecule to a target sequence of a nucleic acid molecule to be depleted, in accordance with the disclosure.
FIGURE 3E schematically illustrates the sample solution of FIGURE 3D after enzymatically extending the capture primer nucleic acid molecule annealed to the target sequence, in accordance with the disclosure.
FIGURE 3F schematically illustrates the sample solution of FIGURE 3E after enzymatically degrading double-stranded sample nucleic acid molecules in the sample solution, in accordance with the disclosure.
FIGURE 3G schematically illustrates melting the sample nucleic acid molecules of the sample solution of FIGURE 2F, in accordance with the disclosure.
FIGURE 3H schematically illustrates the sample solution of FIGURE 3G after removing the capture primer nucleic acid molecules, in accordance with the disclosure.
FIGURE 3I schematically illustrates the sample solution of FIGURE 3H further including PCR primers complementary to a universal adaptor sequence of the sample nucleic acid molecules in the sample solution, in accordance with the disclosure.
FIGURE 3J schematically illustrates the sample solution of FIGURE 3I after PCR amplification of the sample nucleic acid molecules of the sample solution, in accordance with the disclosure.
FIGURE 4 schematically illustrates capture primer nucleic acid molecules, in accordance with an embodiment of the disclosure, bound to Hygro.
FIGURE 5 schematically illustrates capture primer nucleic acid molecules, in accordance with an embodiment of the disclosure, bound to AmpR.
FIGURE 6 schematically illustrates capture primer nucleic acid molecules including a universal adaptor sequence including a polyT sequence, in accordance with an embodiment of the disclosure, bound to AmpR.
FIGURE 7 schematically illustrates capture primer nucleic acid molecules molecules including a universal adaptor sequence including a polyT sequence, in accordance with an embodiment of the disclosure, bound to Hygro.
FIGURE 8 is an image of an electrophoresis gel showing results of an electrophoresis experiment showing enrichment of sample nucleic acid molecules including a target sequence, in accordance with an embodiment of the disclosure.

### DETAILED DESCRIPTION

The present disclosure provides kits and methods for enriching target nucleic acid sequences, such as nucleic acid molecules including the target nucleic acid sequence, and kits and methods for depleting target nucleic acid sequences, such as nucleic acid molecules including the target nucleic acid sequences. The method of the invention is defined according to the claims.

As used herein, the terms "nucleic acid" and "polynucleotides" refer to biopolymers that are made from monomer units referred to as "nucleotides." Typically, each nucleotide is composed of a 5-carbon sugar, a phosphate group, and a nitrogenous base (also referred to as "nucleobase"). The structure of the sugar component typically defines to the type of nucleic acid polymer. The nucleotide monomers link up to form a linear sequence of the nucleic acid polymer. Nucleic acids encompassed by the present disclosure can include deoxyribonucleic acid (DNA), ribonucleic acid (RNA), cDNA or a synthetic nucleic acid known in the art, such as peptide nucleic acid (PNA), glycerol nucleic acid (GNA), threose nucleic acid (TNA), locked nucleic acid (LNA) or other synthetic polymers with nucleotide side chains, or any combination thereof. Nucleic acid molecules can be single stranded or double stranded (with complementary single-stranded polynucleotide chains hybridizing by base pairing of the individual nucleobases). Typically cDNA, RNA, GNA, TNA or LNA are single stranded. DNA can be either double stranded (dsDNA) or single stranded (ssDNA).

Nucleotide subunits of nucleic acids can be naturally occurring, artificial, or modified. As indicated above, nucleotide typically contains a nucleobase, a sugar, and at least one phosphate group. The nucleobase is typically heterocyclic. Suitable nucleobases include the canonical purines and pyrimidines, and more specifically adenine (A), guanine (G), thymine (T) (or typically in RNA, uracil (U) instead of thymine (T)), and cytosine (C). The sugar is typically a pentose sugar. Suitable sugars include, but are not limited to, ribose and deoxyribose. The nucleotide is typically a ribonucleotide or deoxyribonucleotide. The nucleotide typically contains a monophosphate, diphosphate or triphosphate. These are generally referred to herein as nucleotides or nucleotide residues to indicate the subunit. Without specific identification, the term nucleotides, nucleotide residues, and the like, is not intended to imply any specific structure or identity. As indicated above, the nucleic acids of the present disclosure can also include synthetic variants of DNA or RNA. "Synthetic variants" encompasses nucleic acids incorporating known analogs of natural nucleotides/nucleobases that can hybridize to nucleic acids in a manner similar to naturally occurring nucleotides. Exemplary synthetic variants include peptide nucleic acids (PNAs), phosphorothioate DNA, locked nucleic acids, and the like. Modified or synthetic nucleobases and analogs can include, but are not limited to, 5-Br-UTP, 5-Br-dUTP, 5-F-UTP, 5-F-dUTP, 5-propynyl dCTP, 5-propynyl-dUTP, diaminopurine, S2T, 5-fluorouracil, 5-bromouracil, 5-chlorouracil, 5-iodouracil, hypoxanthine, xantine, 4-acetylcytosine, 5-(carboxyhydroxylmethyl)uracil, 5-carboxymethylaminomethyl-2-thiouridine, 5-carboxymethylaminomethyluracil, dihydrouracil, beta-D-galactosylqueosine, inosine, N 6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-D46-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, 2,6-diaminopurine and the like. Persons of ordinary skill in the art can readily determine what base pairings for each modified nucleobase are deemed a base-pair match versus a base-pair mismatch.

### METHODS

The present disclosure provides methods for enriching and/or depleting target nucleic acid sequences, such as target nucleic acid sequences present on sample nucleic acids in a in complex sample solutions comprising sample nucleic acid molecules that do not include the target nucleic acid sequence.

### ENRICHMENT METHODS

The present invention provides a method for enriching a target nucleic acid sequence , the method comprising
(a) introducing to a sample solution, comprising a plurality of sample nucleic acid molecules each comprising a universal adaptor nucleic acid sequence, a capture primer nucleic acid molecule complementary to or partially complementary to a target nucleic acid sequence of one or more sample nucleic acid molecules of the plurality of sample nucleic acid molecules; (b) enzymatically extending the capture primer nucleic acid molecule annealed to the target nucleic acid sequence of the one or more sample nucleic acid molecules; and (c) enzymatically degrading single-stranded sample nucleic acid molecules , to provide an enriched sample solution having a higher proportion of sample nucleic acid molecules comprising the target nucleic acid sequence than the sample solution.

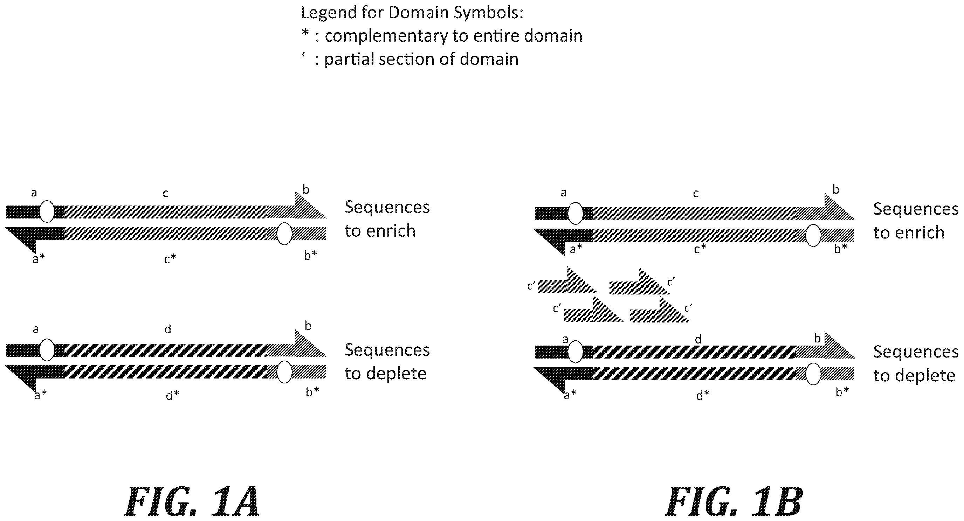

A method for enriching target nucleic acid sequences in accordance with an embodiment of the disclosure will now be described. In that regard, attention is directed to FIGURES 1A-1J, which schematically illustrates a method of enriching a target nucleic acid sequence, in accordance with an embodiment of the disclosure.

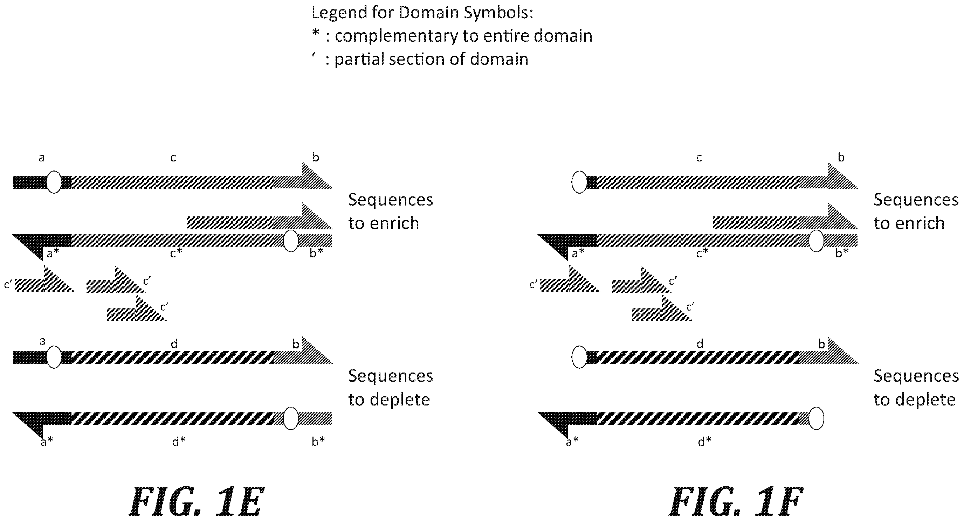

FIGURE 1A schematically illustrates a sample solution including nucleic acid molecules to enrich and nucleic acid molecules to deplete. As shown, the sample solution includes a starting pool of nucleic acid molecules including a double-stranded nucleic acid molecule for enrichment and a double stranded nucleic acid molecule for depletion. While the sample nucleic acid molecules are shown to be double stranded, in an embodiment, the sample nucleic acid molecules include single-stranded sample nucleic acid molecules or a combination of single-stranded and double-stranded sample nucleic acid molecules. The double-stranded nucleic acid molecule for enrichment is shown to include universal adaptor nucleic acid sequences a, a*, b, and b*, and target nucleic acid sequences c and c*. The double-stranded nucleic acid molecule for depletion is shown to include universal adaptor nucleic acid sequences a, a*, b, and b*, and nucleic acid sequences d and d* different from the target nucleic acid sequences c and c*. The universal adaptor nucleic acid sequences a, a*, b, and b*, on both the sample nucleic acid molecules for enrichment and for depletion, are shown to include a common feature, illustrated schematically here as an oval. As discussed further herein with respect to FIGURE 1F, such a common feature is suitable for enzymatic degradation under certain conditions, such as where the universal adaptor nucleic acid sequence is single stranded.

The methods of the present disclosure are suitable to enrich a number of sample solutions comprising nucleic acid molecules. In an embodiment, the sample solution is selected from the group consisting of a WGS library, a WES library, ATAC-seq library, ChIP-seq library, WTS library, Bisulfite-seq library, RNA-seq library, single-cell RNA-seq library, DNA data storage library, or any other library with universal adapters on both ends. The mixture of DNA molecules can be previously amplified or unamplified, generated enzymatically or chemically synthesized. The universal adapters (domain a and domain b*) can include DNA and/or RNA nucleotides. As discussed further herein, at least one of the ribonucleotides may be a guanine. In an embodiment, the universal adaptor nucleic acid sequences present on all or substantially all nucleic acid molecules in the library.

In an embodiment, the sample solution includes double- or single-stranded sample nucleic acid molecules, such as from a WGS library, a WES library, ATAC-seq library, CHIP-seq library, WTS library, Bisulfite-seq library, RNA-seq library, and the like, containing 3' modifications configured to prevent or limit self-annealing and extension. In an embodiment, such 3' modifications include dideoxynucleotides (ddNTPs), inverted 3'dT, or nucleotide sequences that reduce binding energy (e.g. adenine, thymine, or uracil). In one embodiment, the starting sample solution includes double-stranded sample nucleic acid molecules, such as a WGS library, a WES library, ATAC-seq library, CHIP-seq library, WTS library, Bisulfite-seq library, RNA-seq library, and the like, generated by using PCR primers that contain polyT or polyA overhangs on the 5' end.

In an embodiment, the universal adaptor nucleic acid sequences are added through PCR, transposition, reverse transcription, ligation, chemical synthesis, or other known methods to add adapters to DNA sequences, such as discussed further herein with respect to the kits of the present disclosure.

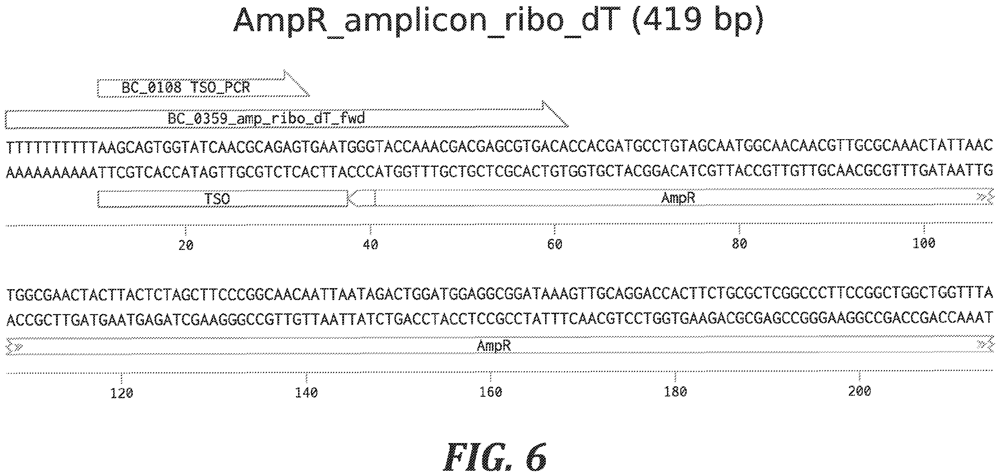

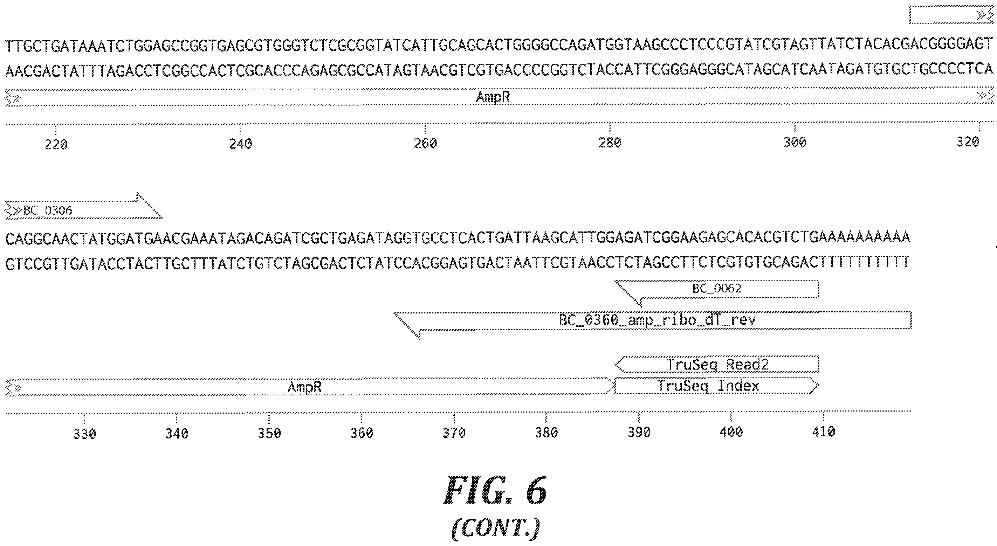

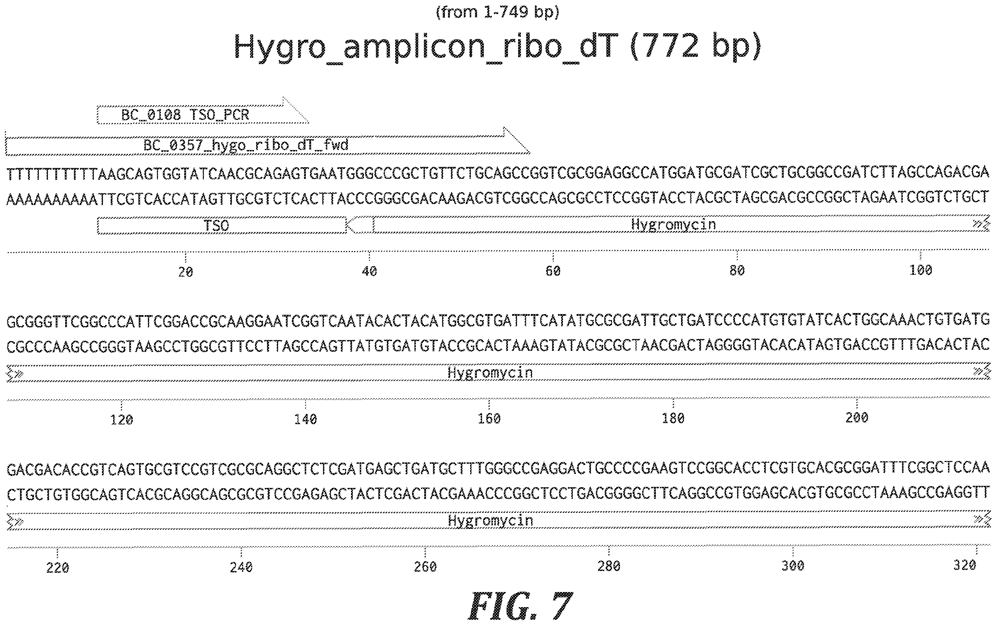

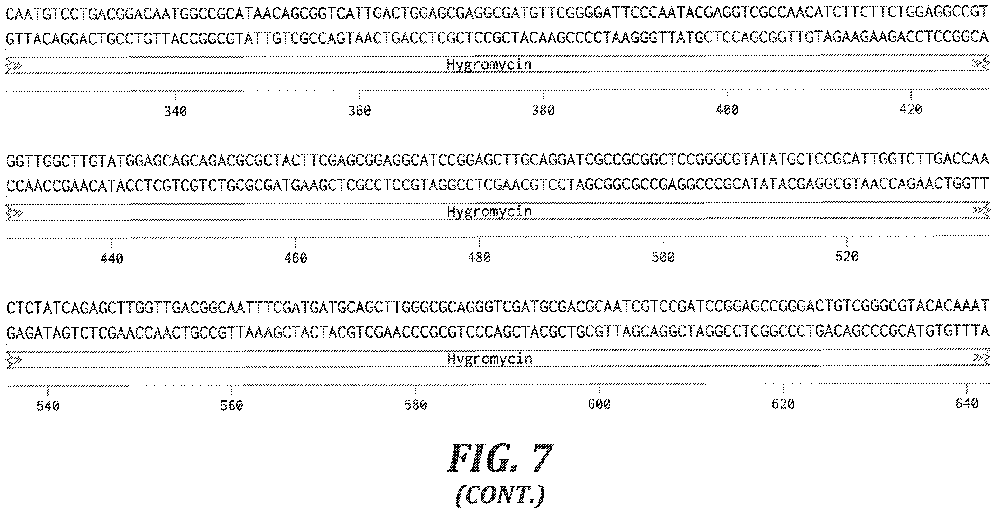

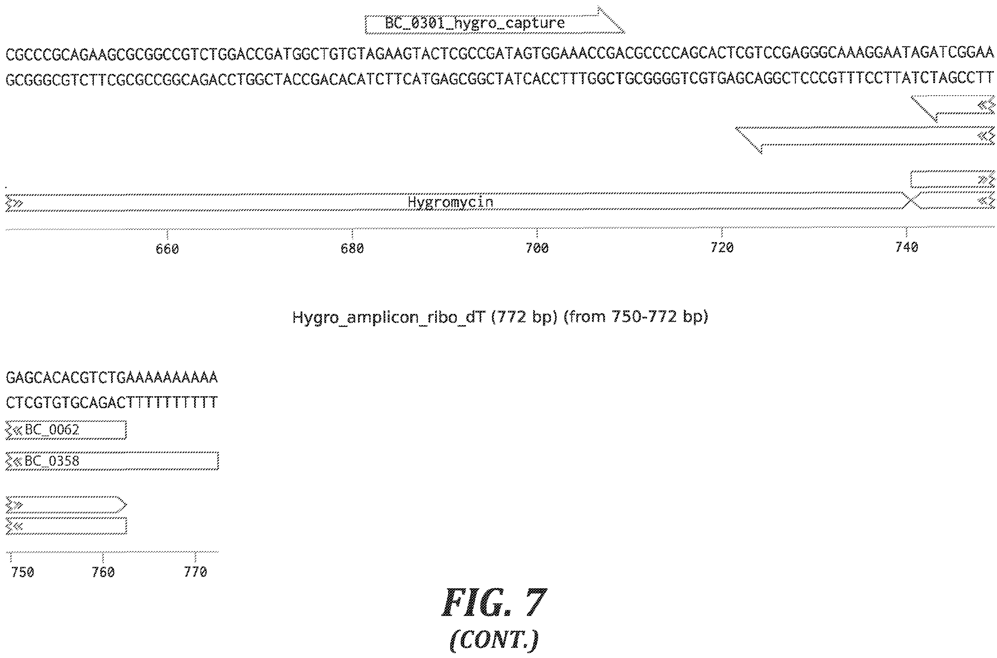

In an embodiment, the universal adaptor nucleic acid sequence includes a nucleic acid sequence adjacent to a 3' end or a 5' end that is configured not to bind to itself, such as in a hairpin configuration, thus avoiding self-priming. In an embodiment, the universal adaptor nucleic acid molecule includes a polyT sequence, a polyA sequence, or a combination thereof. See for example, FIGURES 6 and 7.

In an embodiment, nucleotides in the sample solution include ribonucleotides or deoxynucleotides. In an embodiment, such nucleotides include nucleotides selected from the group consisting of locked nucleic acids, peptide nucleic acids, 2'-O-methyl RNA, 2'-O:-methoxy ethyl RNA, phosphorothioate modified nucleic acids, and the like. Accordingly, in an embodiment, the degradation enzyme discussed further herein, such as, RNase T1, is replaced by a degradation enzyme capable of selectively cleaving the modified ribonucleotide or deoxynucleotide in a single-stranded conformation.

As above, the method includes introducing to the sample solution one or more capture primer nucleic acid molecule(s) complementary to or partially complementary to a target nucleic acid sequence of one or more sample nucleic acid molecules of the plurality of sample nucleic acid molecules. FIGURE 1B schematically illustrates the sample solution of FIGURE 1A further including capture primer nucleic acid molecules c', in accordance with an embodiment of the disclosure.

As above, the capture primer nucleic acid molecule is complementary to or partially complementary to the target nucleic acid sequence. In an embodiment, the capture primer nucleic acid molecule is partially complementary to the target nucleic acid sequence. In an embodiment, the capture primer nucleic acid molecule comprises a number of bases that are not complementary to the target nucleic acid sequence, such as in a range of 1 to 5. In an embodiment, the capture primer nucleic acid molecule is greater than or equal to 90% complementary to the target nucleic acid sequence. Such partially complementary capture primer nucleic acid molecules are, nevertheless, configured to bind with target nucleic acid sequences, such as depending upon the annealing temperatures and/or other reaction conditions described herein.

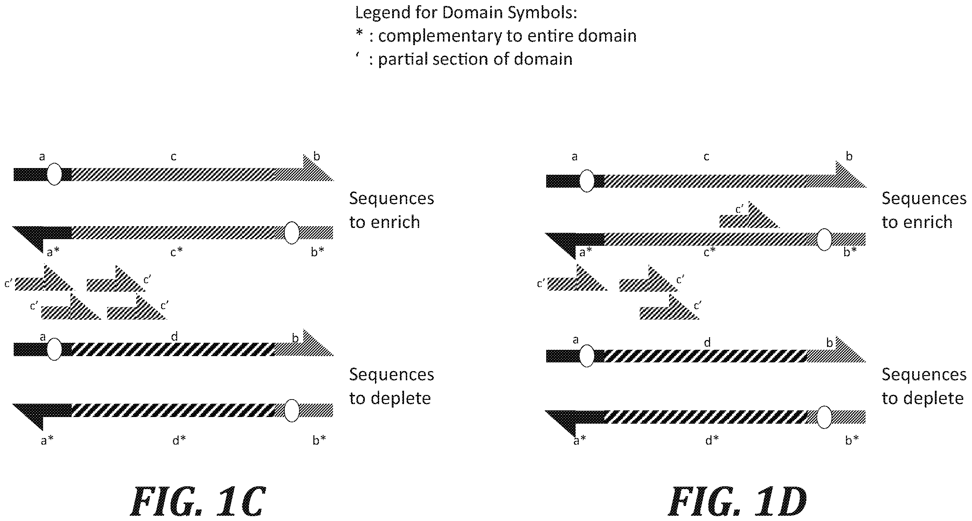

In an embodiment, the method includes maintaining a temperature of the sample solution at or above a melting temperature of the plurality of sample nucleic acid molecules. FIGURE 1C schematically illustrates the sample solution of FIGURE 1B after melting the nucleic acid molecules to enrich and to deplete, in accordance with an embodiment of the disclosure. In an embodiment, the melting temperature is greater than or equal to 95°C. At the melting temperature of the plurality of sample nucleic acid molecules the temperature of the sample solution is sufficient to completely or partially break Watson-Crick bonding between sample nucleic acid molecules, thereby increasing the number of single-stranded or partially single-stranded sample nucleic acid molecules in the sample solution. As shown, such melting exposes target nucleic acid sequences c and c*, as well as nucleic acid sequences d and d*, to bonding with other nucleic acid sequences, such as the capture primer nucleic acid molecules, c'.

In an embodiment, the method includes maintaining the sample solution at about or below an annealing temperature of the capture primer nucleic acid molecule suitable to anneal the capture primer nucleic acid molecule to the target nucleic acid sequence. Such an annealing temperature is generally suitable to anneal at least a portion of the capture primer nucleic acid molecules to the target nucleic acid sequence. In an embodiment, the annealing temperature is in a range of about 50°C to about 72°C. FIGURE 1D schematically illustrates the sample solution of FIGURE 1C after annealing a capture primer nucleic acid molecule c' to a target sequence c* of a nucleic acid molecule to be enriched, in accordance with an embodiment of the disclosure. In the illustrated embodiment, one of the capture primer nucleic acid molecules c' is bound to the target nucleic acid sequence c* of a sample nucleic acid molecule to be enriched.

In an embodiment, the capture primer nucleic acid molecule is configured to be primarily single stranded at the annealing temperature. In this regard, the capture primer nucleic acid molecule is single stranded a majority of the time at the annealing temperature, and is, therefore, configured to bind to the target nucleic acid sequence a majority of the time. In an embodiment, the capture primer nucleic acid molecule is configured to be primarily at least partially double stranded at the annealing temperature. In this regard, the capture primer nucleic acid molecule is in a configuration suitable for binding to a target nucleic acid sequence less than a majority of the time at the annealing temperature. Thus, binding of such a double-stranded capture primer nucleic acid molecule to a target nucleic acid sequence is generally more selective than for single-stranded capture primer nucleic acid molecules.

In an embodiment, the capture primer nucleic acid molecule further comprises a second capture primer nucleic acid molecule complementary to or partially complementary to a first capture primer nucleic acid molecule. Such double-stranded capture primer nucleic acid molecules are generally double stranded at the annealing temperature and are, thus, less often configured to bind to a target nucleic acid sequence. In this regard, such double-stranded capture primer nucleic acid molecules are configured to bind more selectively to target nucleic acid sequences.

In an embodiment, the capture primer nucleic acid molecule is complementary to or partially complementary to a second target nucleic acid sequence of one or more second sample nucleic acid molecules of the plurality of sample nucleic acid molecules, wherein the second target nucleic acid sequence is different than the target nucleic acid sequence. In this regard, by maintaining the sample solution at or at about an annealing temperature of the capture primer nucleic acid molecule, the capture primer nucleic acid molecules may bind to various target nucleic acid sequences. As discussed further herein with respect to FIGURES 1E and 1F, sample nucleic acid molecules comprising various target sequences complementary to or partially complementary to the capture primer nucleic acid molecules may be enzymatically extended and protected from degradation.

In an embodiment, the capture primer nucleic acid molecule comprises a phosphorothioate linkage. In an embodiment, the phosphorothioate linkage is disposed between a base at a 3' end of the capture primer nucleic acid molecule and a base immediately adjacent to the base at the 3' end. Such phosphorothioate linkages are configured to resist 3' exonuclease activity, such as those present in proof reading polymerases.

As above, the sample nucleic acid molecules include a universal adaptor nucleic acid sequence. In an embodiment, the universal adaptor nucleic acid sequence of the plurality of sample nucleic acid molecules comprises an adaptor tag nucleic acid sequence. In an embodiment, the adaptor tag nucleic acid sequence defines a unique nucleic acid sequence. Such a unique sequence can be used to determine an origin of the sample nucleic acid molecules, such as a cell, tissue, or suspension of origin, where such unique nucleic acid sequences have different sequences from another adaptor tag nucleic acid sequence used to tag sample nucleic acid molecules in other samples, such as in other cells, tissues, or suspensions of cells.

Such adaptor tag nucleic acid sequences are suitable for counting a number of nucleic acid molecules in a sample, such as through sequencing the sample solution. In an embodiment, each adaptor tag nucleic acid molecule includes a number of degenerate bases suitable for counting amplified sample nucleic acid molecules after a nucleic acid amplification reaction.

In an embodiment, an annealing temperature of the capture primer nucleic acid molecule and the second target nucleic acid sequence is relatively close to the annealing temperature of the capture primer nucleic acid molecule and the target nucleic acid sequence, such that by maintaining the sample solution at the annealing temperature of the capture primer nucleic acid molecule and the target nucleic acid sequence, at least some of the capture primer nucleic acid molecules bind to the second target nucleic acid sequence. Accordingly, in an embodiment, the capture primer nucleic acid molecule and the second target nucleic acid sequence have a second annealing temperature in a range of about 1 °C to about 5 °C of the annealing temperature.

In an embodiment, the sample solution is maintained at temperatures that are near, but not necessarily precisely at, the annealing temperature. In this regard, the binding specificity of the capture primer nucleic acid molecules is varied, allowing the capture primer nucleic acid molecules to bind, for example, to a number of target nucleic acid sequences having relatively similar sequences, and thus enriching a number of different sample nucleic acid molecules. Accordingly, in an embodiment, maintaining the sample solution at about or below an annealing temperature of the capture primer nucleic acid molecule comprises maintaining the sample solution at a temperature within a range of about 1 °C to about 5 °C of the annealing temperature of the capture primer nucleic acid molecule.

As above, the methods include enzymatically extending the capture primer nucleic acid molecule annealed to the target nucleic acid sequence of the one or more sample nucleic acid molecules. In an embodiment, enzymatically extending the capture primer nucleic acid molecule comprises introducing to the sample solution an extension enzyme configured to extend the capture primer nucleic acid molecule annealed to the target nucleic acid sequence. FIGURE 1E schematically illustrates the sample solution of FIGURE 1D after enzymatically extending the capture primer nucleic acid molecule c' annealed to the target sequence c*, in accordance with an embodiment of the disclosure. As shown, the nucleic acid sequence annealed to the target nucleic acid sequence c* is shown extended to also bind with the universal adaptor nucleic acid sequence b*. As discussed further herein, by binding to the universal adaptor nucleic acid sequence, the extended capture primer nucleic acid molecule inhibits enzymatic degradation of the double-stranded sample nucleic acid molecule.

The extension enzyme can include any enzyme configured to enzymatically extend the capture primer nucleic acid molecule annealed to another nucleic acid molecule. In an embodiment, the extension enzyme is selected from the group consisting of a polymerase, a reverse transcriptase, and combinations thereof.

In an embodiment, enzymatically extending the capture primer nucleic acid molecule comprises maintaining the sample solution at about an extension temperature of the extension enzyme suitable for enzymatic extension by the extension enzyme of the capture primer nucleic acid molecule annealed to the target nucleic acid sequence. Such an extension temperature may be the same as or different from the annealing temperature. In an embodiment, the extension temperature is in a range of about 68°C to about 72°C.

The methods of the present disclosure include enzymatically degrading certain nucleic acid molecules of the sample solution to provide an enriched sample solution having a higher proportion of sample nucleic acid molecules comprising the target nucleic acid sequence(s) than the sample solution. In an embodiment, such enzymatic degradation includes enzymatically degrading single-stranded sample nucleic acid molecules. As discussed above with respect to FIGURES 1D and 1E, sample nucleic acid molecules including nucleic acid sequences complementary to or partially complementary to the capture primer nucleic acid molecules may be generally double-stranded. In this regard, by enzymatically degrading single-stranded nucleic acid molecules, such as in conjunction with other steps such as amplifying the intact sample nucleic acid molecules, the sample solution is enriched for such nucleic acid molecules including target nucleic acid sequences.

In an embodiment, enzymatically degrading single-stranded sample nucleic acid molecules comprises introducing to the sample solution a degradation enzyme configured to degrade a single-stranded nucleic acid molecule comprising the universal adaptor nucleic acid sequence. In an embodiment, the degradation enzyme is introduced to the sample solution after enzymatically extending the capture primer nucleic acid molecule. In an embodiment, wherein the degradation enzyme is introduced to the sample solution before enzymatically extending the capture primer nucleic acid molecule. In such an embodiment, the degradation enzyme may not be active at, for example, at the extension temperature, and, therefore, does not or does not substantially degrade single-stranded nucleic acid molecules at the extension temperature. Rather, in an embodiment, the degradation enzyme is active a temperature lower than the extension temperature.

In an embodiment, enzymatically degrading single-stranded sample nucleic acid molecules comprises maintaining the temperature of the sample solution at a degradation temperature of the degradation enzyme. In an embodiment, the degradation temperature is below the annealing temperature. In an embodiment, the degradation temperature is below the extension temperature. In an embodiment, the degradation temperature is less than or equal to about 60°C.

In an embodiment, the degradation temperature is an active temperature of the degradation enzyme. Accordingly, by maintaining the sample solution at or at about the degradation, the degradation enzyme is active, such as active in degrading single-stranded nucleic acid molecules. In an embodiment, the degradation enzyme is inactive at a temperature chosen from the extension temperature, the melting temperature, the annealing temperature, and combinations thereof. In this regard, the degradation enzyme does not or does not substantially enzymatically degrade single-stranded nucleic acid molecules in the sample solution, such as before enzymatic extension of annealed capture primer nucleic acid molecules annealed to the target nucleic acid sequences.

In an embodiment, the degradation enzyme is active at the degradation temperature after being inactive at a temperature above the degradation temperature, such as the extension temperature. In this regard, in an embodiment, the degradation enzyme is configured to preferentially or selectively degrade sample nucleic acid molecules, such as single-stranded sample nucleic acid molecules, after having been inactive at a temperature above the degradation temperature. Without wishing to be bound by theory, it is believed that the degradation enzyme is inactive above the active temperature, such as when the degradation enzyme takes on an inactive conformation, and that the degradation further becomes active when the degradation enzyme assumes an active configuration when the temperature of the sample solution is maintained in an active range.

In an embodiment, enzymatically degrading the single-stranded sample nucleic acid molecules includes degrading a portion of the universal adaptor nucleic acid sequence disposed on the single-stranded sample nucleic acid molecules. FIGURE 1F schematically illustrates the sample solution of FIGURE 1E after enzymatically degrading the single-stranded sample nucleic acid molecules, in accordance with an embodiment of the disclosure. In the illustrated embodiment, the degradation enzyme is shown to have enzymatically degraded a portion of the single-stranded nucleic acid molecule including the universal adaptor sequence b*, formerly including the targeted portion of the universal adaptor nucleic acid sequence (illustrated here as an oval). This is in contrast to the double-stranded sample nucleic acid, which includes the target nucleic acid sequence c* and has been enzymatically extended by the extension enzyme. In this regard, the double-stranded sample nucleic acid is shown to have an intact universal adaptor nucleic acid sequence b*.

In an embodiment, the universal adaptor nucleic acid sequence is entirely single stranded. In this regard, the universal adaptor nucleic acid sequence is not base paired with other nucleic acid sequences, such as on separate nucleic acid molecules. In an embodiment, the universal adaptor nucleic acid sequence is only partially single stranded. In an embodiment, the universal adaptor nucleic acid sequence is single stranded at one or more nucleotides configured to be enzymatically degraded by the degradation enzyme when single stranded.

Enzymatic degradation of the single-stranded sample nucleic acid molecules can include a number of forms of degradation configured, for example, to make the degraded sample nucleic acid unsuitable for nucleic acid amplification reactions, such as those including the universal adaptor nucleic acid molecules. In an embodiment, enzymatically degrading the single-stranded sample nucleic acid molecules includes cleaving a backbone of the universal adaptor nucleic acid molecule of the single-stranded sample nucleic acid molecules. In an embodiment, enzymatically degrading the single-stranded sample nucleic acid molecules includes digesting a portion of the universal adaptor nucleic acid molecule of the single-stranded sample nucleic acid molecules.

As above, in an embodiment, the degradation enzyme is configured to enzymatically degrade single-stranded nucleic acid molecules, such as single-stranded sample nucleic acid molecules. In an embodiment, the degradation enzyme is a ribonuclease. In an embodiment, the degradation enzyme is an endonuclease. In an embodiment, the endonuclease is an endoribonuclease. In an embodiment, the endoribonuclease is selected from the group consisting of Rnase T1, Rnase A, and combinations thereof.

In an embodiment, the degradation enzyme is Rnase T1. In an embodiment, the degradation enzyme is according to SEQ ID NO. 14. In an embodiment, the degradation has a sequence homology to SEQ ID NO. 14 greater than 90%, greater than 95%, or greater than 99%. In an embodiment, the universal adaptor nucleic acid sequence comprises a riboguanine. In an embodiment, the universal adaptor nucleic acid sequence comprises a plurality of riboguanines. Rnase T1 selectively degrades single-stranded riboguanines, and, accordingly, where the universal adaptor nucleic acid sequence includes one or more riboguanines, the Rnase T1 degradation enzyme is configured to degrade the universal adaptor nucleic acid sequence, such as when the sample solution is maintained at an active temperature of Rnase T1.

In an embodiment, the degradation enzyme is Rnase A. In an embodiment, the degradation enzyme is according to SEQ ID NO. 15. In an embodiment, the degradation has a sequence homology to SEQ ID NO. 15 greater than 90%, greater than 95%, or greater than 99%. In an embodiment, the universal adaptor nucleic acid sequence comprises bases selected from the group consisting of a ribocytosine, a ribouracil, and combinations thereof. In an embodiment, the universal adaptor nucleic acid sequence comprises a plurality of ribocytosines, a plurality of ribouracils, and combinations thereof. Rnase A selectively degrades single-stranded ribocytosines and ribouracils (such as at salt concentrations above 300 mM), and accordingly, where the universal adaptor nucleic acid sequences includes one or more ribocytosines and/or ribouracils, the Rnase A degradation enzyme is configured to degrade the universal adaptor nucleic acid sequence, such as when the sample solution is maintained at an active temperature of Rnase A.

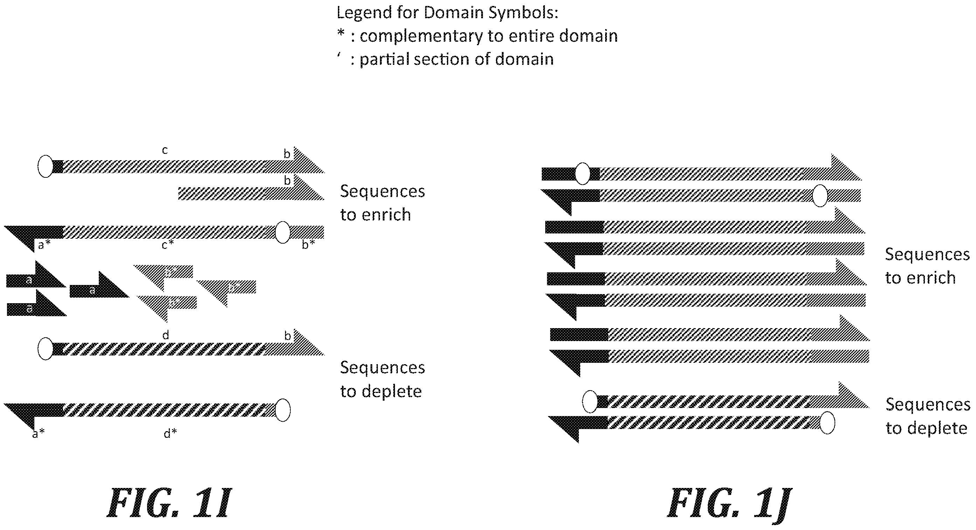

In an embodiment, the method of the present disclosure includes repeating enzymatically extending the capture primer nucleic acid molecule and enzymatically degrading single-stranded sample nucleic acid molecules. By repeating enzymatic extension and enzymatic degradation, the extension enzyme, capture primer nucleic acid molecules, and degradation enzyme can be used one or more additional times to selectively degrade sample nucleic acid molecules that do not include a target nucleic acid sequence. As above, in an embodiment, such degradation includes degrading the universal adaptor nucleic acid sequence, which can be later used in a nucleic acid amplification reaction. As discussed further herein with respect to FIGURE 1I and 1J, sequences that include intact universal adaptor nucleic acid sequences are preferentially enriched.

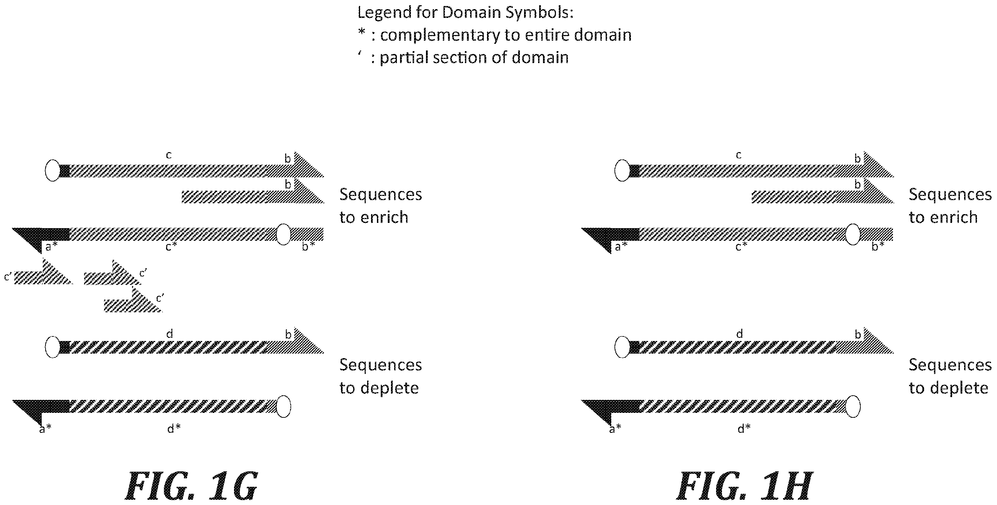

In an embodiment, the method further includes maintaining the temperature of the sample solution at or above a melting temperature of the plurality of sample nucleic acid molecules and the capture primer nucleic acid molecule, such as after enzymatically extending the capture primer nucleic acid molecule and enzymatically degrading single-stranded sample nucleic acid molecules. In this regard, the sample solution including sample nucleic acid molecules having enzymatically degraded or intact universal adaptor nucleic acid sequences are single stranded and, thus configured for further enzymatic extension and degradation. FIGURE 1G schematically illustrates melting the nucleic acid molecules of the sample solution of FIGURE 1F, in accordance with an embodiment of the disclosure.

In an embodiment, the method of the present disclosure includes purifying the plurality of sample nucleic acid molecules in the enriched sample solution. FIGURE 1H schematically illustrates the sample solution of FIGURE 1G after removing the capture primer nucleic acid molecules, in accordance with an embodiment of the disclosure. Such purification can include, for example, purification with SPRI beads and the like. In an embodiment, purifying the plurality of sample nucleic acid molecules in the enriched sample solution comprises removing reagents chosen from capture primer nucleic acid molecules, enzymes, and combinations thereof from the enriched sample solution. Such purification of the sample solution can simplify sequencing data based on the sample solution, such as by reducing the number of nucleic acid molecules present in the sample solution and, thereby, decreasing an amount of sequencing data based on the sample solution, particularly reducing an amount of sequencing data not related to target nucleic acid sequences.

In an embodiment, the method of the present disclosure includes amplifying sample nucleic acid molecules after enzymatic degradation of single-stranded nucleic acid molecules. Accordingly, in an embodiment the method includes introducing a plurality of amplification primer nucleic acid molecules to the enriched sample solution. In an embodiment, the amplification primer nucleic acid molecules of the plurality of amplification primer nucleic acid molecules are complementary to the universal adaptor nucleic acid sequence. FIGURE 1I schematically illustrates the sample solution of FIGURE 1H further including polymerase chain reaction (PCR) primers a* and b. As shown, the PCR primers are complementary to the universal adaptor sequences a* and b of the sample nucleic acid molecules in the sample solution, in accordance with an embodiment of the disclosure.

In an embodiment, the method includes performing a nucleic acid amplification reaction on the plurality of sample nucleic acid molecules in the enriched sample solution with the plurality of amplification primer nucleic acid molecules to provide an amplified enriched sample solution. FIGURE 1J schematically illustrates the sample solution of FIGURE 1I after PCR amplification of the sample nucleic acid molecules of the sample solution, in accordance with an embodiment of the disclosure. As shown, the sample solution includes a greater proportion of sample nucleic acid molecules including the target sequences c and c* than nucleic acid sequences d and d*.

As discussed above and shown in FIGURE 1J, because at least some of the universal adaptor nucleic acid sequences of the sample nucleic acid molecules are degraded, these degraded sample nucleic acid molecules will not participate in the nucleic acid amplification reaction, and thus the amplified enriched sample solution will contain a lower proportion of such sample nucleic acid molecules. In this regard, in an embodiment, performing the nucleic acid amplification reaction on the plurality of sample nucleic acid molecules in the enriched sample solution does not or does not substantially amplify sample nucleic acid molecules that have been degraded by the degradation enzyme.

In an embodiment, the method includes performing one or more enzymatic reactions on the amplified enriched sample to solution to prepare the enriched sample solution for sequencing, such as a next-generation sample preparation. Accordingly, in an embodiment, the method of the present disclosure includes performing a reaction on the amplified enriched sample solution chosen from a nucleic acid fragmentation reaction, enzymatic end repair, A tailing, adaptor ligation, polymerase chain reaction, and combinations thereof.

In an embodiment, the method of the present disclosure includes sequencing nucleic acid molecules in the enriched sample solution. In an embodiment, sequencing nucleic acid molecules in the enriched sample solution comprises generating sample nucleic acid information based upon the plurality of sample nucleic acid molecules in the enriched sample solution. As above, in certain embodiment, the universal adaptor nucleic acid molecules include an adaptor tag nucleic acid molecule. In an embodiment, sequencing nucleic acid molecules in the enriched sample solution comprises generating adaptor tag nucleic sequence information based on the adaptor tag nucleic acid sequences.

### DEPLETION METHODS

Also disclosed is a method for depleting a target nucleic acid sequence. The method may comprise (a) introducing to a sample solution, comprising a plurality of sample nucleic acid molecules each comprising a universal adaptor nucleic acid sequence comprising ribonucleotides, a capture primer nucleic acid molecule complementary or partially complementary to a target nucleic acid sequence of one or more sample nucleic acid molecules of the plurality of sample nucleic acid molecules; (b) enzymatically extending the capture primer nucleic acid molecule annealed to the target nucleic acid sequence of the one or more sample nucleic acid molecules; and (c) enzymatically cleaving double-stranded ribonucleic acid molecules of the sample nucleic acid molecules, to provide a depleted sample solution having a lower proportion of sample nucleic acid molecules comprising the target nucleic acid sequence than the sample solution.

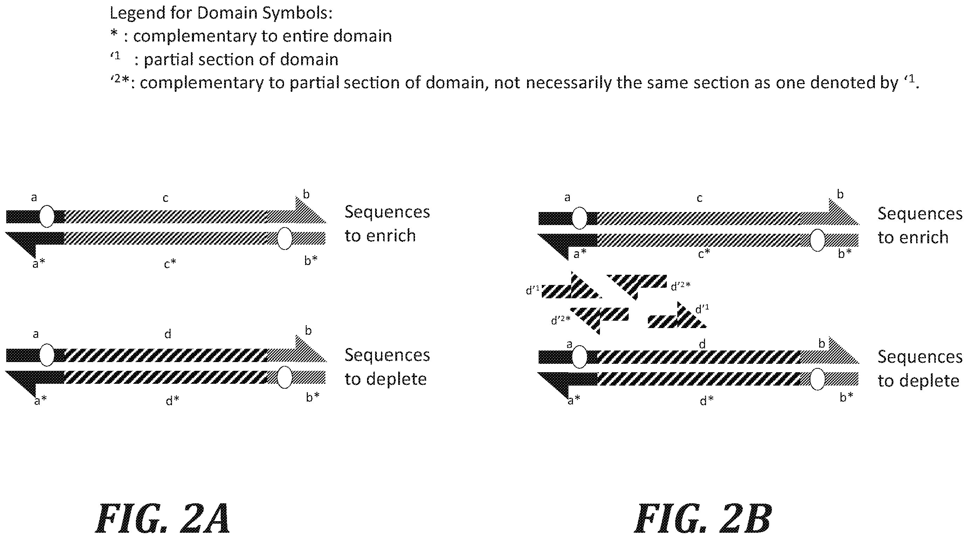

A method for depleting target nucleic acid sequences in accordance with the disclosure will now be described. In that regard, attention is directed to FIGURES 2A-2J, which schematically illustrates a method of depleting a target nucleic acid sequence, in accordance with the disclosure.

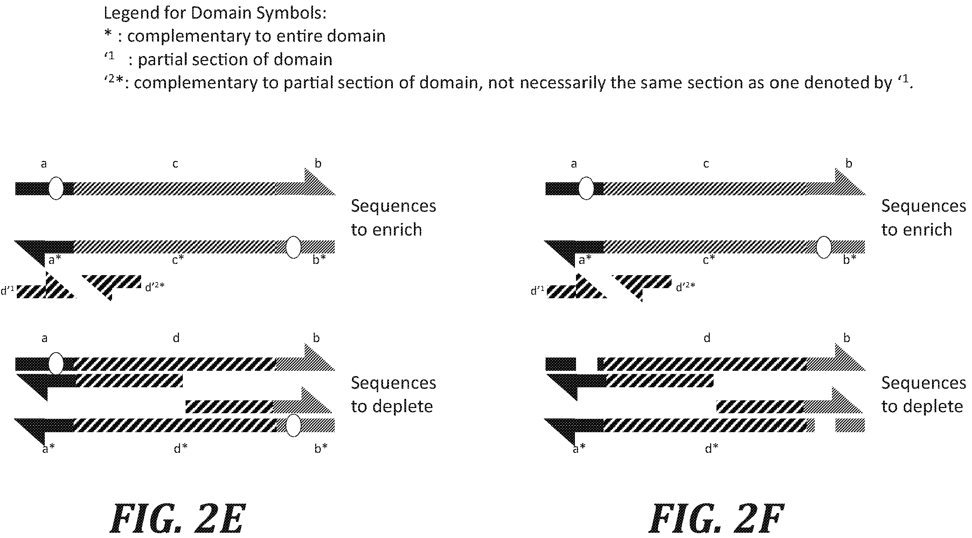

FIGURE 2A schematically illustrates a sample solution including nucleic acid molecules to enrich and nucleic acid molecules to deplete. As shown, the sample solution includes a starting pool of nucleic acid molecules including a double-stranded nucleic acid molecule for enrichment and a double stranded nucleic acid molecule for depletion. The double-stranded sample nucleic acid molecule for enrichment is shown to include universal adaptor nucleic acid sequences a, a*, b, and b*, and target nucleic acid sequences c and c*. The double-stranded nucleic acid molecule for depletion is shown to include universal adaptor nucleic acid sequences a, a*, b, and b*, and target nucleic acid sequences d and d* different from the nucleic acid sequences c and c*. The universal adaptor nucleic acid sequences a, a*, b, and b*, on both the nucleic acid molecules for enrichment and for depletion, are shown to include a common feature, illustrated schematically here as an oval. As discussed further herein with respect to FIGURE 2F, such a common feature is suitable for enzymatic degradation under certain conditions, such as where the universal adaptor nucleic acid sequence is double stranded.

The methods are suitable to enrich a number of sample solutions comprising nucleic acid molecules. The sample solution may be selected from the group consisting of a WGS library, a WES library, ATAC-seq library, ChIP-seq library, WTS library, Bisulfite-seq library, RNA-seq library, single-cell RNA-seq library, DNA data storage library, or any other library with universal adapters on both ends. The mixture of DNA molecules can be previously amplified or unamplified, generated enzymatically or chemically synthesized. The universal adapters (domain a and domain b*) can include DNA and/or RNA nucleotides. As discussed further herein, at least one of the ribonucleotides may be a guanine. The universal adaptor nucleic acid sequences may be present on all or substantially all nucleic acid molecules in the library.

The sample solution may include double- or single-stranded sample nucleic acid molecules, such as from a WGS library, a WES library, ATAC-seq library, CHIP-seq library, WTS library, Bisulfite-seq library, RNA-seq library, and the like, containing 3' modifications configured to prevent or limit self-annealing and extension. In an instance, such 3' modifications include dideoxynucleotides (ddNTPs), inverted 3'dT, or nucleotide sequences that reduce binding energy (e.g. adenine, thymine, or uracil).

The starting sample solution may include double-stranded sample nucleic acid molecules, such as a WGS library, a WES library, ATAC-seq library, CHIP-seq library, WTS library, Bisulfite-seq library, RNA-seq library, and the like, generated by using PCR primers that contain polyT or polyA overhangs on the 5' end.

In an instance, the universal adaptor nucleic acid sequences are added through PCR, transposition, reverse transcription, ligation, chemical synthesis, or other known methods to add adapters to DNA sequences, such as discussed further herein with respect to the kits of the present disclosure.

The universal adaptor nucleic acid sequence may include a nucleic acid sequence adjacent to a 3' end or a 5' end that is configured not to bind to itself, such as in a hairpin configuration, thus avoiding self-priming. In an instance, the universal adaptor nucleic acid molecule includes a polyT sequence, a polyA sequence, or a combination thereof. See for example, FIGURES 6 and 7.

Nucleotides in the sample solution may include ribonucleotides or deoxynucleotides. In an instance, such nucleotides include nucleotides selected from the group consisting of locked nucleic acids, peptide nucleic acids, 2'-O-methyl RNA, 2'-O:-methoxy ethyl RNA, phosphorothioate modified nucleic acids, and the like. Accordingly, the degradation enzyme, such as RNase HII, may be replaced with a degradation enzyme configured to selectively cleave the modified ribonucleotide or deoxynucleotide in a double-stranded conformation. In an instance, the sample nucleic acid molecules include methylated DNA and the degradation enzyme includes a restriction enzyme that specifically cleaves methylated (or hemimethylated) double stranded DNA.

The method may include introducing to the sample solution one or more capture primer nucleic acid molecule(s) complementary to or partially complementary to a target nucleic acid sequence of one or more sample nucleic acid molecules of the plurality of sample nucleic acid molecules. FIGURE 2B schematically illustrates the sample solution of FIGURE 2A further including capture primer nucleic acid molecules d'¹ and d'²*, in accordance with the disclosure. As shown, the capture primer nucleic acid molecules d'¹ and d'²* are complementary or partially complementary to a target nucleic acid sequences d* and d on the sample nucleic acid molecules for depletion, rather than the sample nucleic acid molecules for enrichment.

In an instance, the capture primer nucleic acid molecule is complementary to or partially complementary to the target nucleic acid sequence. In an instance, the capture primer nucleic acid molecule is partially complementary to the universal adaptor nucleic acid sequence. The capture primer nucleic acid molecule may comprise a number of bases that are not complementary to the universal adaptor nucleic acid sequence, such as in a range of 1 to 5. In an instance, the capture primer nucleic acid molecule is greater than or equal to 90% complementary to the universal adaptor sequence. Such partially complementary capture primer nucleic acid molecules are, nevertheless, configured to bind with target nucleic acid sequences, such as depending upon the annealing temperatures and/or other reaction conditions described herein.

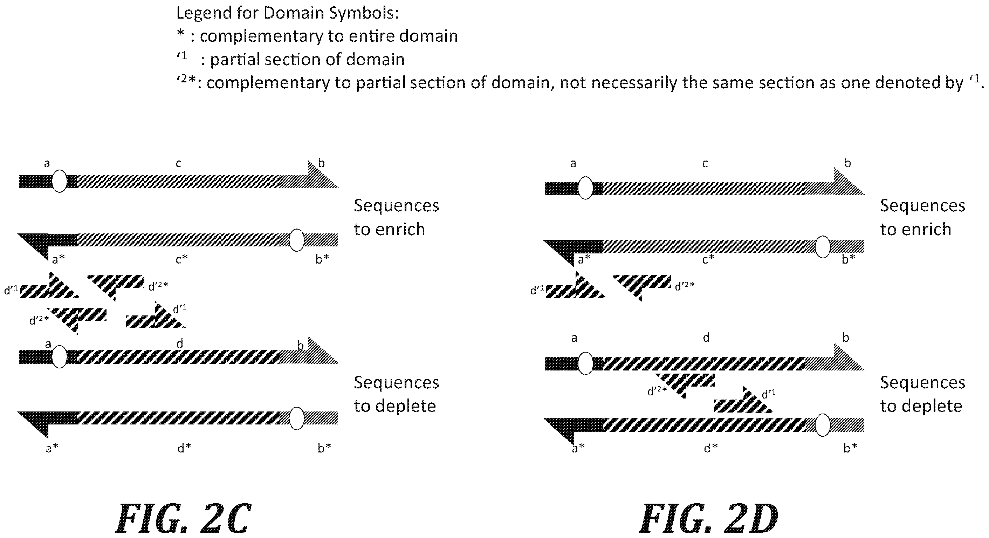

The method may include maintaining a temperature of the sample solution at or above a melting temperature of the plurality of sample nucleic acid molecules. FIGURE 2C schematically illustrates the sample solution of FIGURE 2B after melting the sample nucleic acid molecules to enrich and to deplete, in accordance with the disclosure. In an instance, the melting temperature is greater than or equal to 95°C. At melting temperature of the plurality of sample nucleic acid molecules the temperature of the sample solution is sufficient to completely or partially break Watson-Crick bonding between sample nucleic acid molecules, thereby increasing the number of single-stranded or partially single-stranded sample nucleic acid molecules in the sample solution. As shown, such melting exposes target nucleic acid sequences d and d*, as well as nucleic acid sequences c and c*, to bonding with other nucleic acid sequences, such as the capture primer nucleic acid molecules d'¹ and d'²*.

The method may include maintaining the sample solution at about or below an annealing temperature of the capture primer nucleic acid molecule suitable to anneal the capture primer nucleic acid molecule to the target nucleic acid sequence. Such an annealing temperature is generally suitable to anneal at least a portion of the capture primer nucleic acid molecules to the target nucleic acid sequence. In an instance, the annealing temperature is in a range of about 50°C to about 72°C. FIGURE 2D schematically illustrates the sample solution of FIGURE 2C after annealing capture primer nucleic acid molecules d'¹ and d'²*, to a target sequences d* and d of sample nucleic acid molecules to be depleted, in accordance with the disclosure. In the illustrated method, capture primer nucleic acid molecules d'¹ and d'²* are bound to the target nucleic acid sequences d* and d of sample nucleic acid molecules to be depleted.

In an instance, the capture primer nucleic acid molecule is configured to be primarily single stranded at the annealing temperature. In this regard, the capture primer nucleic acid molecule is single stranded a majority of the time at the annealing temperature, and is, therefore, configured to bind to the target nucleic acid sequence a majority of the time. In an instance, the capture primer nucleic acid molecule is configured to be primarily at least partially double stranded at the annealing temperature. In this regard, the capture primer nucleic acid molecule is in a configuration suitable for binding to a target nucleic acid sequence less than a majority of the time at the annealing temperature. Thus, binding of such a double-stranded capture primer nucleic acid molecule to a target nucleic acid sequence is generally more selective than for single-stranded capture primer nucleic acid molecules.

In an instance, the capture primer nucleic acid molecule further comprises a second capture primer nucleic acid molecule complementary to or partially complementary to a first capture primer nucleic acid molecule. Such double-stranded capture primer nucleic acid molecules are generally double stranded at the annealing temperature and are, thus, less often configured to bind to a target nucleic acid sequence. In this regard, such double-stranded capture primer nucleic acid molecules are configured to bind more selectively to target nucleic acid sequences.

In an instance, the capture primer nucleic acid molecule is complementary to or partially complementary to a second target nucleic acid sequence of one or more second sample nucleic acid molecules of the plurality of sample nucleic acid molecules, wherein the second target nucleic acid sequence is different than the target nucleic acid sequence. In this regard, by maintaining the sample solution at or at about an annealing temperature of the capture primer nucleic acid molecule, the capture primer nucleic acid molecules may bind to various target nucleic acid sequences. As discussed further herein with respect to FIGURES 2E and 2F, sample nucleic acid molecules comprising various target sequences complementary to or partially complementary to the capture primer nucleic acid molecules may be enzymatically extended and marked for degradation.

In an instance, the capture primer nucleic acid molecule comprises a phosphorothioate linkage. The phosphorothioate linkage may be disposed between a base at a 3' end of the capture primer nucleic acid molecule and a base immediately adjacent to the base at the 3' end. Such phosphorothioate linkages are configured to resist 3' exonuclease activity, such as those present in proof reading polymerases.

As above, the sample nucleic acid molecules include a universal adaptor nucleic acid sequence. In an instance, the universal adaptor nucleic acid sequence of the plurality of sample nucleic acid molecules comprises an adaptor tag nucleic acid sequence. In an instance, the adaptor tag nucleic acid sequence defines a unique nucleic acid sequence. Such unique sequence can be used to determine an origin of the sample nucleic acid molecules, such a cell, tissue, or suspension of origin, where such unique nucleic acid sequences have different sequences from another adaptor tag nucleic acid sequence used to tag sample nucleic acid molecules in other samples, such as in other cells, tissues, or suspensions of cell.

Such adaptor tag nucleic acid sequences are suitable for counting a number of nucleic acid molecules in a sample, such as through sequencing the sample solution.

Each adaptor tag nucleic acid molecule may include a number of degenerate bases suitable for counting amplified sample nucleic acid molecules after a nucleic acid amplification reaction.

In an instance, an annealing temperature of the capture primer nucleic acid molecule and the second target nucleic acid sequence is relatively close to the annealing temperature of the capture primer nucleic acid molecule and the target nucleic acid sequence, such that by maintaining the sample solution at the annealing temperature of the capture primer nucleic acid molecule and the target nucleic acid sequence, at least some of the capture primer nucleic acid molecules bind to the second target nucleic acid sequence. Accordingly, in an instance, the capture primer nucleic acid molecule and the second target nucleic acid sequence have a second annealing temperature in a range of about 1 °C to about 5 °C of the annealing temperature.

In an instance, the sample solution is kept at temperatures that are near, but not necessarily precisely at, the annealing temperature. In this regard, the binding specificity of the capture primer nucleic acid molecules is varied, allowing the capture primer nucleic acid molecules to bind, for example, to a number of target nucleic acid sequences having relatively similar sequences, and thus depleting a number of different sample nucleic acid molecules. Accordingly, maintaining the sample solution at about or below an annealing temperature of the capture primer nucleic acid molecule may comprise maintaining the sample solution at a temperature within a range of about 1 °C to about 5 °C of the annealing temperature of the capture primer nucleic acid molecule.

As above, the method may include enzymatically extending the capture primer nucleic acid molecule annealed to the target nucleic acid sequence of the one or more sample nucleic acid molecules. FIGURE 2E schematically illustrates the sample solution of FIGURE 2D after enzymatically extending the capture primer nucleic acid molecules d'¹ and d'²* annealed to the target sequences d* and d, in accordance with
the disclosure. As shown, the capture primer nucleic acid molecules d'¹ and d'²* are annealed to the target sequences d* and d on the sample nucleic acid molecules to be depleted. As also shown, the nucleic acid sequence annealed to the target nucleic acid sequence d and d* are shown extended to also bind with the universal adaptor nucleic acid sequences a and b*. As discussed further herein, by binding to the universal adaptor nucleic acid sequences a and b*, the extended capture primer nucleic acid molecule activates enzymatic degradation of the double-stranded sample nucleic acid molecule.

The extension enzyme can include any enzyme configured to enzymatically extend the capture primer nucleic acid molecule annealed to another nucleic acid molecule.

The extension enzyme may be selected from the group consisting of a polymerase, a reverse transcriptase, and combinations thereof.

Enzymatically extending the capture primer nucleic acid molecule may comprise maintaining the sample solution at about an extension temperature of the extension enzyme suitable for enzymatic extension by the extension enzyme of the capture primer nucleic acid molecule annealed to the target nucleic acid sequence. Such an extension temperature may be the same as or different from the annealing temperature. In an instance, the annealing temperature is in a range of about 50°C to about 72°C.

As above, the methods may include enzymatically cleaving double-stranded ribonucleic acid molecules of the sample nucleic acid molecules. FIGURE 2F schematically illustrates the sample solution of FIGURE 2E after enzymatically degrading double-stranded nucleic acid molecules, in accordance with the disclosure. In the illustrated method, the universal adaptor nucleic acid sequences a and b* bound to the enzymatically extended capture primer nucleic acid molecules are degraded. In this regard, the ovals of the capture primer nucleic acid molecules are shown to be degraded. As discussed further herein with respect to FIGURE 2F, such degradation can include cleaving or degrading a backbone of the universal adaptor nucleic acid sequence of the double-stranded sample nucleic acid molecules.

In the illustrated method, the degradation enzyme is shown to have enzymatically degraded a portion of the double-stranded nucleic acid molecule including the universal adaptor sequences a and b*, including the targeted portion of the universal adaptor nucleic acid sequence (illustrated here as an oval). Sample nucleic acid molecules including the target nucleic acid sequences d and d* have enzymatically degraded universal adaptor sequences a and b*. This is in contrast to the single-stranded sample nucleic acid, which includes the nucleic acid sequence c and c*, which have intact universal adaptor sequences. In this regard, the single-stranded sample nucleic acid is shown to have an intact universal adaptor nucleic acid sequence.

Enzymatic degradation of the double-stranded sample nucleic acid molecules can include a number of forms of degradation configured, for example, to make the degraded sample nucleic acid unsuitable for nucleic acid amplification reactions, such as those including the universal adaptor nucleic acid molecules. In an instance, enzymatically degrading the double-stranded sample nucleic acid molecules includes cleaving a backbone of the universal adaptor nucleic acid molecule of the double-stranded sample nucleic acid molecules. Enzymatically cleaving the double-stranded sample nucleic acid molecules may include degrading a portion of the universal adaptor nucleic acid sequence disposed on the double-stranded sample nucleic acid molecules.

Enzymatically cleaving the double-stranded sample nucleic acid molecules may include cleaving a backbone of the universal adaptor nucleic acid sequence of the double-stranded sample nucleic acid molecules. In an instance, enzymatically cleaving the double-stranded sample nucleic acid molecules includes digesting a portion of the universal adaptor nucleic acid sequence of the double-stranded sample nucleic acid molecules.

Enzymatically degrading double-stranded sample nucleic acid molecules may comprise maintaining the temperature of the sample solution at a degradation temperature of the degradation enzyme. The degradation temperature may be below the annealing temperature, below the extension temperature , or is less than or equal to about 60°C.

The degradation temperature may be an active temperature of the degradation enzyme. Accordingly, by maintaining the sample solution at or at about the degradation, the degradation enzyme is active, such as active in degrading double-stranded nucleic acid molecules. In an instance, the degradation enzyme is inactive at a temperature chosen from the extension temperature, the melting temperature, the annealing temperature, and combinations thereof. In this regard, the degradation enzyme does not or does not substantially enzymatically degrade double-stranded nucleic acid molecules in the sample solution, such as before enzymatic extension of annealed capture primer nucleic acid molecules annealed to the target nucleic acid sequences.

The degradation enzyme may be active at the degradation temperature after being inactive at a temperature above the degradation temperature, such as the extension temperature. In this regard, the degradation enzyme may be configured to preferentially or selectively degrade sample nucleic acid molecules, such as double-stranded sample nucleic acid molecules, after having been inactive at a temperature above the degradation temperature. Without wishing to be bound by theory, it is believed that the degradation enzyme is inactive above the active temperature, such as when the degradation enzyme takes on an inactive conformation, and that the degradation further becomes active when the degradation enzyme assumes an active configuration when the temperature of the sample solution is maintained in an active range.

As above, the degradation enzyme may be configured to enzymatically degrade double-stranded nucleic acid molecules, such as double-stranded sample nucleic acid molecules. In an instance, the degradation enzyme is not a restriction endonuclease. The degradation enzyme may be a ribonuclease, or an endonuclease. In an instance, the endonuclease is an endoribonuclease. and may be selected from the group consisting of Rnase HII, RNase H, Rnase III, and combinations thereof.

In an instance, the degradation enzyme is Rnase HII. In an instance, the degradation enzyme is according to SEQ ID NO. 16. In an instance, the degradation has a sequence homology to SEQ ID NO. 16 greater to 90%, greater than 95%, or greater than 99%.

In an instance, the degradation enzyme is Rnase H. In an instance, the degradation enzyme is according to SEQ ID NO. 17. In an instance, the degradation has a sequence homology to SEQ ID NO. 17 greater to 90%, greater than 95%, or greater than 99%.

In an instance, the degradation enzyme is Rnase III. In an instance, the degradation enzyme is according to SEQ ID NO. 18. In an instance, the degradation has a sequence homology to SEQ ID NO. 18 greater to 90%, greater than 95%, or greater than 99%.

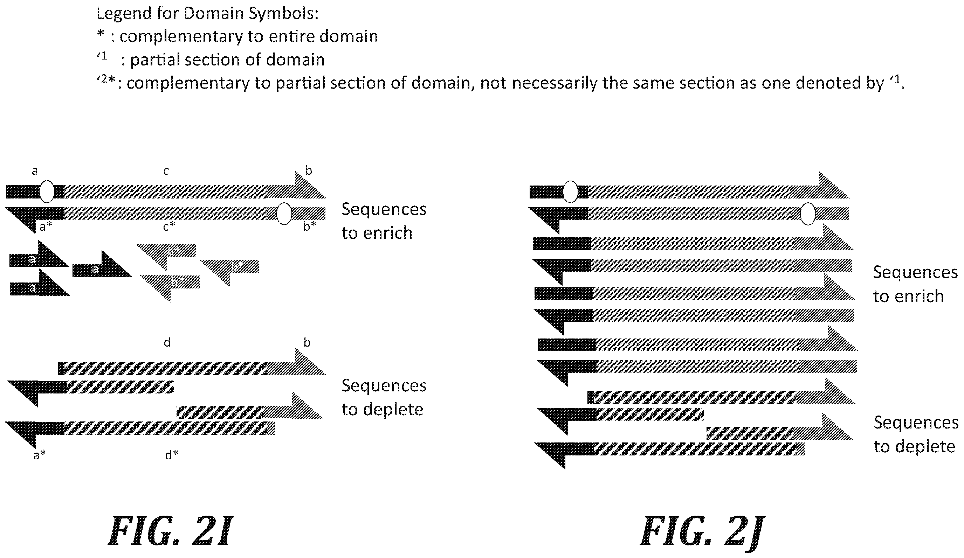

The method may include repeating enzymatically extending the capture primer nucleic acid molecule and enzymatically degrading double-stranded sample nucleic acid molecules. By repeating enzymatic extension and enzymatic degradation, the extension enzyme, capture primer nucleic acid molecules, and degradation enzyme can be used one or more additional times to selectively degrade sample nucleic acid molecules that include a target nucleic acid sequence, such as target sequences d and d*. As above, such degradation may include degrading the universal adaptor nucleic acid sequence, which can be later used in a nucleic acid amplification reaction. As discussed further herein with respect to FIGURE 2I and 2J, nucleic acid sequences that include intact universal adaptor nucleic acid sequences are preferentially enriched. Accordingly, by enzymatically degrading additional sample nucleic acid sequences that have target nucleic acid sequences, sample nucleic acid molecules that do not have the target nucleic acid sequences a configured not to take part in such selective or preferential enrichment.

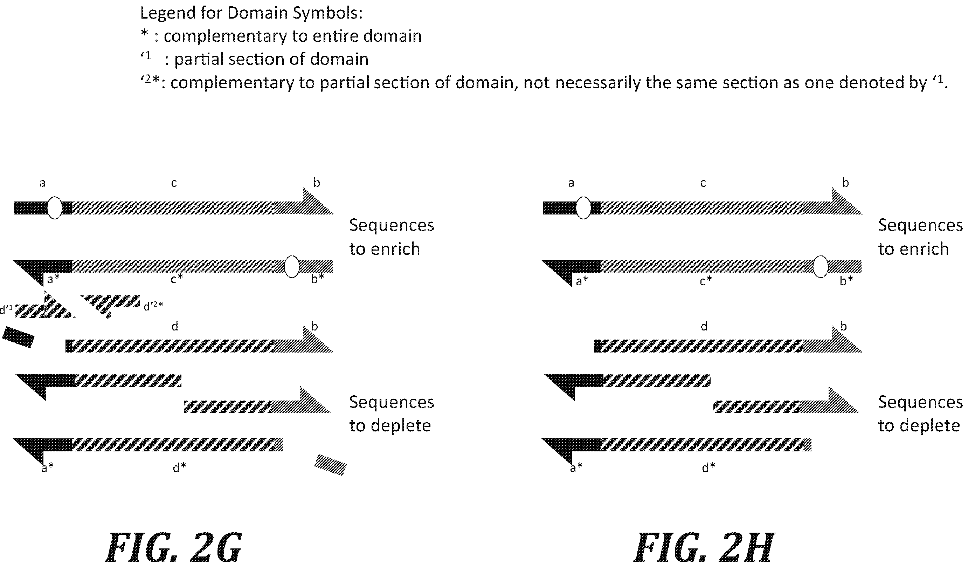

The method may further include maintaining the temperature of the sample solution at or above a melting temperature of the plurality of sample nucleic acid molecules and the capture primer nucleic acid molecule, such as after enzymatically extending the capture primer nucleic acid molecule and enzymatically degrading double-stranded sample nucleic acid molecules. In this regard, the sample solution including sample nucleic acid molecules having enzymatically degraded or intact universal adaptor nucleic acid sequences are single stranded and, thus configured to later bind with capture primer nucleic acid molecules. FIGURE 2G schematically illustrates melting the nucleic acid molecules of the sample solution of FIGURE 2F, in accordance with the disclosure.

The method may include purifying the plurality of sample nucleic acid molecules in the depleted sample solution. FIGURE 2H schematically illustrates the sample solution of FIGURE 2G after removing the capture primer nucleic acid molecules d'¹ and d'²*, in accordance with the disclosure. Such purification can include, for example, purification with SPRI beads and the like. In an instance, purifying the plurality of sample nucleic acid molecules in the depleted sample solution comprises removing reagents chosen from capture primer nucleic acid molecules, enzymes, and combinations thereof from the depleted sample solution. Such purification of the sample solution can simplify sequencing data based on the sample solution, such as by reducing the number of nucleic acid molecules present in the sample solution and, thereby, decreasing an amount of sequencing data based on the sample solution, particularly reducing an amount of sequencing data not related to target nucleic acid sequences.

The method may include amplifying sample nucleic acid molecules after enzymatic degradation of double-stranded nucleic acid molecules. Accordingly, the method may include introducing a plurality of amplification primer nucleic acid molecules to the depleted sample solution. In an instance, the amplification primer nucleic acid molecules of the plurality of amplification primer nucleic acid molecules are complementary to the universal adaptor nucleic acid sequence. FIGURE 2I schematically illustrates the sample solution of FIGURE 2H further including polymerase chain reaction (PCR) primers a and b*. As shown, the PCR primers are complementary to the universal adaptor sequences a* and b of the nucleic acid molecules in the sample solution, in accordance with the disclosure.

The method may include performing a nucleic acid amplification reaction on the plurality of sample nucleic acid molecules in the depleted sample solution with the plurality of amplification primer nucleic acid molecules to provide an amplified depleted sample solution. FIGURE 2J schematically illustrates the sample solution of FIGURE 2I after PCR amplification of the nucleic acid molecules of the sample solution, in accordance with the disclosure. As shown, the sample solution includes a greater proportion of sample nucleic acid molecules including nucleic acid sequences c and c* than samples nucleic acid molecules including the target nucleic acid sequences d and d*.

As discussed above and shown in FIGURE 2J, because at least some of the universal adaptor nucleic acid sequences of the sample nucleic acid molecules are degraded, these degraded sample nucleic acid molecules will not participate in the nucleic acid amplification reaction, and thus the amplified depleted sample solution will contain a lower proportion of such sample nucleic acid molecules. In this regard, in an instance, performing the nucleic acid amplification reaction on the plurality of sample nucleic acid molecules in the depleted sample solution does not or does not substantially amplify sample nucleic acid molecules that have been degraded by the degradation enzyme. Accordingly, the amplified depleted sample solution comprises a greater proportion of nucleic acid molecules that include sequences c or c* than d or d* compared to the original sample solution shown in FIGURE 2A.

The method may include performing one or more enzymatic reactions on the amplified depleted sample to solution to prepare the depleted sample solution for sequencing, such as a next-generation sample preparation. Accordingly,
the method may include performing a reaction on the amplified depleted sample solution chosen from a nucleic acid fragmentation reaction, enzymatic end repair, A tailing, adaptor ligation, polymerase chain reaction, and combinations thereof.

The method may include sequencing nucleic acid molecules in the depleted sample solution. In an instance, sequencing nucleic acid molecules in the depleted sample solution comprises generating sample nucleic acid information based upon the plurality of sample nucleic acid molecules in the depleted sample solution. As above, the universal adaptor nucleic acid molecules may include an adaptor tag nucleic acid molecule. In an instance, sequencing nucleic acid molecules in the depleted sample solution comprises generating adaptor tag nucleic sequence information based on the adaptor tag nucleic acid sequences.

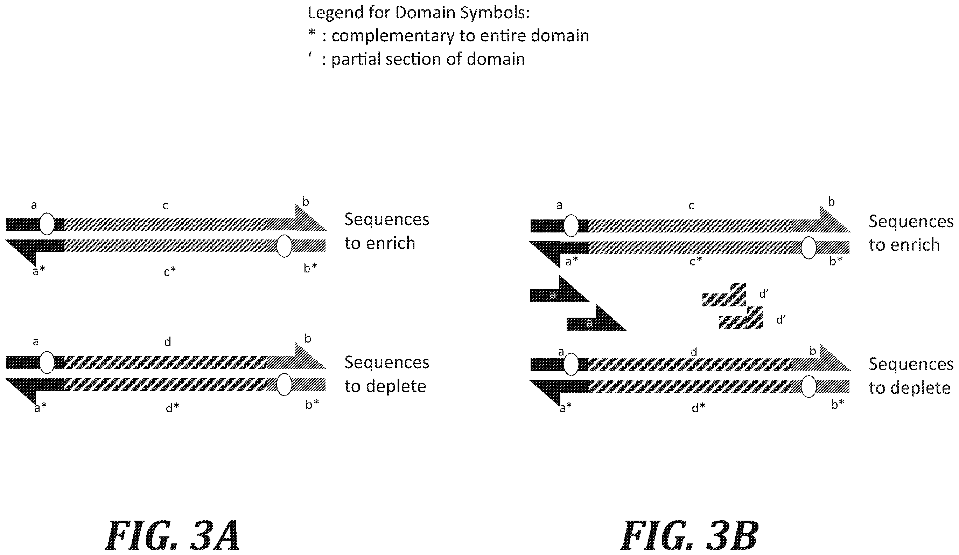

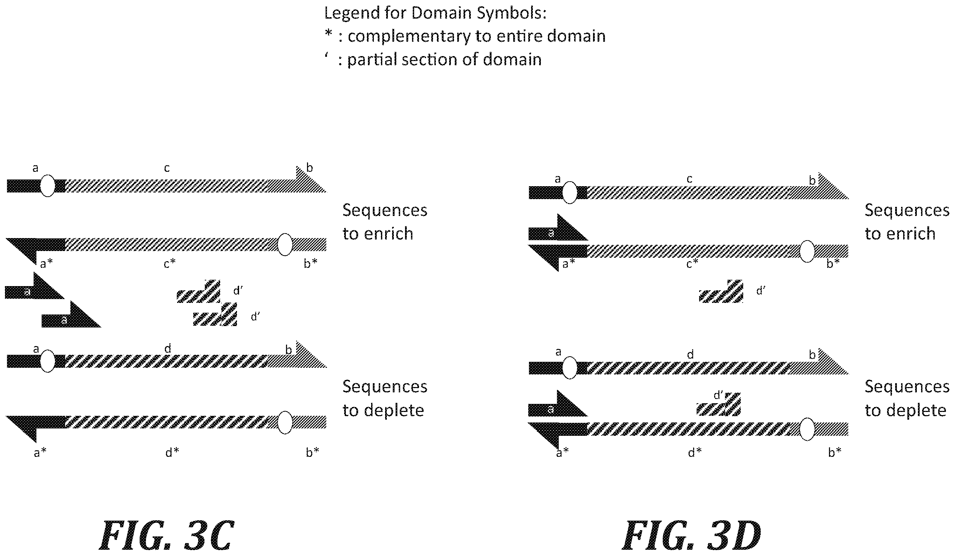

The capture primer nucleic acid molecule may be a blocked capture primer nucleic acid molecule. In that regard, attention is directed to FIGURES 3A-3J, in which a method in accordance with the disclosure is illustrated. FIGURES 3A-3D are analogous to FIGURES 1A-1D, described elsewhere herein, except that the capture primer nucleic acid molecules include capture primer nucleic acid molecule d', which is a blocked capture primer nucleic acid molecule. In that regard, in an instance, the blocked capture primer nucleic acid molecule d' is configured to block enzymatic extension at a 3' end of the blocked capture primer nucleic acid molecule d' by an extension enzyme. As shown, the sample solution further includes a non-blocked capture primer nucleic acid molecule a.

The blocked capture primer nucleic acid molecule may include an inverted nucleic acid, or may include
one or more overhanging adenines or thymines at a 3' end.

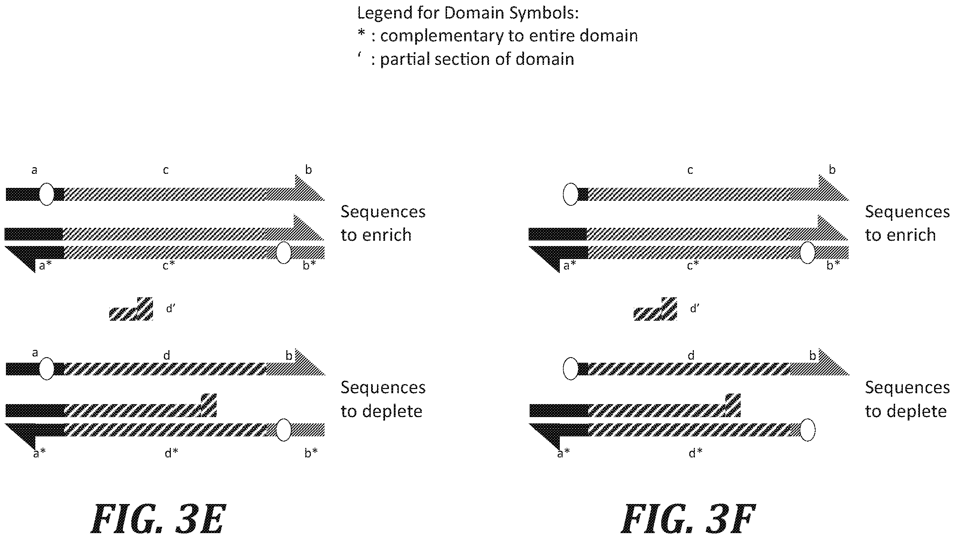

As shown in FIGURE 3E, enzymatic extension where the blocked capture primer nucleic acid molecule is annealed to target nucleic acid sequence d*, the extension enzyme is unable to extend past the blocked capture primer nucleic acid molecule, whereas on other sample nucleic acid molecules, the extension enzyme has successfully extended across the whole molecule, such as the sample nucleic acid molecule for enrichment, which does not include the target nucleic acid sequence d*.

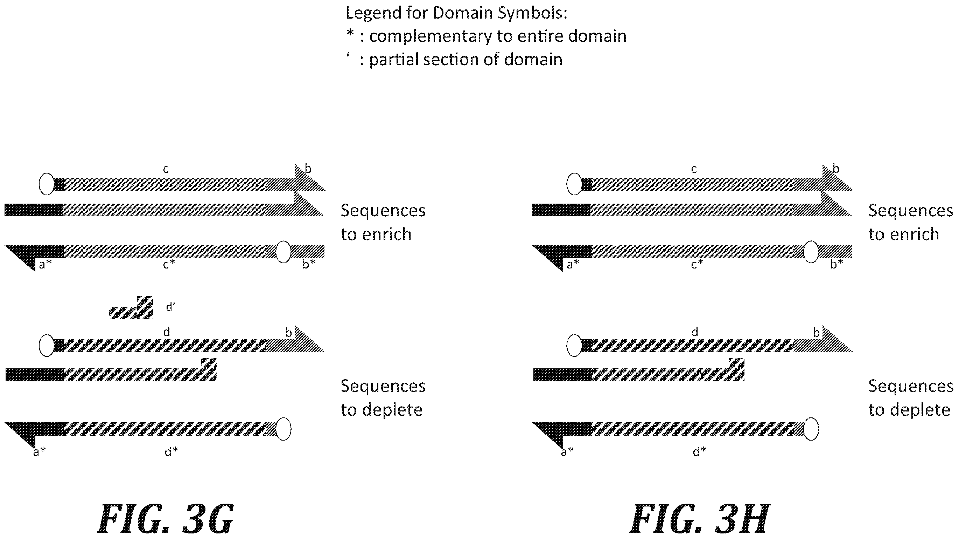

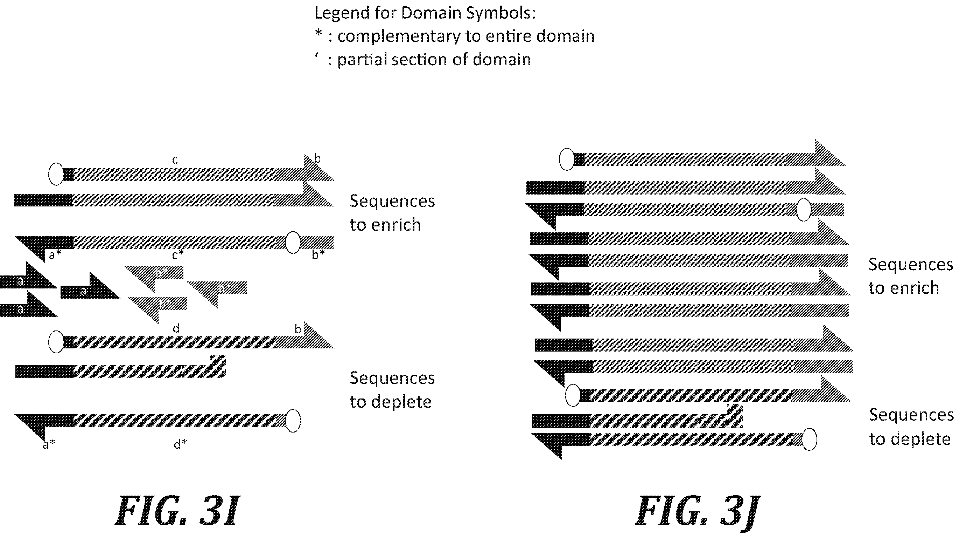

As shown in FIGURE 3F, the degradation enzyme has enzymatically degraded the single-stranded universal adaptor molecule of the sample nucleic acid to be depleted. In this regard, as the sample solution is subsequently melted (FIGURE 3G), purified (FIGURE 3H), and amplified (FIGURES 3I and 3J), molecules including the target nucleic acid sequence d* are depleted and the sample solution is shown to have a higher proportion of sample nucleic acid molecules having sequences c and c* than the target nucleic acid sequences d and d*. The sample solution is, thus, depleted of sample nucleic acid molecules having the target nucleic acid sequence.

While blocked capture primer nucleic acid molecules are shown to deplete sample nucleic acid molecules in conjunction with degradation enzymes configured to degrade single-stranded nucleic acid molecules, blocked capture primer nucleic acid molecules can be used in conjunction with degradation enzymes configured to degrade double-stranded sample nucleic acid molecules to enrich for sample nucleic acid molecules having a target nucleic acid sequence complementary to the blocked capture primer nucleic molecules, in accordance with the disclosure. KITS

Also disclosed are kits including reagents for enriching and/or depleting target nucleic target nucleic acid sequences, such as target nucleic acid sequences present in complex sample solutions comprising nucleic acid molecules that do not include the target nucleic acid sequence.

### ENRICHMENT KITS

The present disclosure provides a kit for enriching sample nucleic acid molecules including a target nucleic acid sequence. The kit may include a capture primer nucleic acid molecule complementary to or partially complementary to a target sequence; and a degradation enzyme configured to degrade a single-stranded nucleic acid molecule.

As above, the kit includes a capture primer nucleic acid molecule. The capture primer nucleic acid molecule may be perfectly complementary to a target nucleic acid sequence , or may be partially complementary to one or more target nucleic acid molecules. As discussed further herein, the capture primer nucleic acid molecules can be at least partially complementary to a number of target nucleic acid sequences, and, thus, the kits of the present disclosure are configured to enrich sample nucleic acid molecules having a number of different target nucleic acid sequences, depending upon the reaction conditions in which they are deployed.

As discussed further herein with respect to FIGURE 1D, the capture primer nucleic acid molecules can be single stranded, at least partially double stranded, or double stranded, such as at an annealing temperature between the capture primer nucleic acid molecule and its target nucleic acid sequence.

The capture primer nucleic acid molecule may comprise a phosphorothioate linkage. The phosphorothioate linkage may be disposed between a base at a 3' end of the capture primer nucleic acid molecule and a base immediately adjacent to the base at the 3' end. Such phosphorothioate linkages are configured to resist 3' exonuclease activity, such as those present in proof reading polymerases.

The kit may further include a plurality of universal adaptor nucleic acid molecules configured to couple to a sample nucleic acid molecule. As discussed further herein with respect to the methods of the present disclosure, the universal adaptor nucleic acid molecules are suitable for use in a nucleic acid amplification reaction.

The universal adaptor nucleic acid molecule may comprise a riboguanine, such as where the degradation enzyme is Rnase T1. The universal adaptor nucleic acid molecule may comprise a ribocytosine, a ribouracil, or combinations thereof, such as where the degradation enzyme is Rnase A.

The universal adaptor nucleic acid molecule may include a nucleic acid sequence adjacent to a 3' end or a 5' end that is configured not to bind to itself, such as in a hairpin configuration, thus avoiding self-priming. The universal adaptor nucleic acid molecule may include a polyT sequence, a polyA sequence, or a combination thereof.

The kit may further comprise reagents for coupling the universal adaptor nucleic acid molecule to a sample nucleic acid molecule. The kit may comprise reagents selected from the group consisting of a transposase loaded with an oligonucleotide comprising a universal adaptor nucleic acid molecule; a restriction endonuclease, an oligonucleotide or oligonucleotide complex comprising a universal adaptor nucleic acid molecule, an oligonucleotide or oligonucleotide complex comprising a T7 promoter, an antibody or antibody fragment against a transcription factor, and combinations thereof.

The kits of the present instance include a degradation enzyme. The degradation enzyme may be configured to degrade a single-stranded nucleic acid molecule or may be configured to degrade single-stranded nucleic acid molecules comprising the universal adaptor nucleic acid molecule.

The degradation enzyme may be a ribonuclease, or
an endonuclease. The endonuclease may be an endoribonuclease , and may be selected from the group consisting of Rnase T1, Rnase A, and combinations thereof.

In an instance, the degradation enzyme is Rnase T1. In an instance, the degradation enzyme is according to SEQ ID NO. 14. In an instance, the degradation has a sequence homology to SEQ ID NO. 14 greater to 90%, greater than 95%, or greater than 99%. In an instance, the universal adaptor nucleic acid sequence comprises a riboguanine. In an instance, the universal adaptor nucleic acid sequence comprises a plurality of riboguanines. Rnase T1 selectively degrades single-stranded riboguanines, and, accordingly, where the universal adaptor nucleic acid sequence includes one or more riboguanines, the Rnase T1 degradation enzyme is configured to degrade the universal adaptor nucleic acid sequence, such as when the sample solution is maintained at an active temperature of Rnase T1.

In an instance, the degradation enzyme is Rnase A. In an instance, the degradation enzyme is according to SEQ ID NO. 15. In an instance, the degradation has a sequence homology to SEQ ID NO. 15 greater to 90%, greater than 95%, or greater than 99%. In an instance, the universal adaptor nucleic acid sequence comprises bases selected from the group consisting of a ribocytosine, a ribouracil, and combinations thereof.

In an instance, the universal adaptor nucleic acid sequence comprises a plurality of ribocytosines, a plurality of ribouracils, and combinations thereof. Rnase A selectively degrades single-stranded ribocytosines and ribouracils (such as at salt concentrations above 300 mM), and accordingly, where the universal adaptor nucleic acid sequences includes one or more ribocytosines and/or ribouracils, the Rnase A degradation enzyme is configured to degrade the universal adaptor nucleic acid sequence, such as when the sample solution is maintained at an active temperature of Rnase A.

The degradation enzyme may be inactive in degrading single-stranded nucleic acid molecules above an active temperature range; and active in degrading single-stranded nucleic acid molecules within the active temperature range after having been inactive. As discussed further herein, the degradation enzyme may be inactive at elevated temperatures, such as at an enzymatic extension temperature, but is active once the temperature of a sample solution is lowered after having been elevated.

The kit may further comprise an extension enzyme configured to extend a capture primer nucleic acid molecule annealed to the target nucleic acid sequence.

The extension enzyme may be selected from the group consisting of a polymerase, a reverse transcriptase, and combinations thereof.

The kit may further comprise instructions for enriching a target nucleic acid sequence, such as in a sample comprising sample nucleic acid molecules.

The kit may comprise instructions for enriching sample nucleic acid molecules including a target nucleic acid sequence. The instructions may comprise instructions comprising: (a) introducing to a sample solution, comprising a plurality of sample nucleic acid molecules each comprising a universal adaptor nucleic acid sequence, a capture primer nucleic acid molecule complementary to or partially complementary to a target nucleic acid sequence of one or more sample nucleic acid molecules of the plurality of sample nucleic acid molecules; (b) enzymatically extending the capture primer nucleic acid molecule annealed to the target nucleic acid sequence of the one or more sample nucleic acid molecules; and (c) enzymatically degrading single-stranded sample nucleic acid molecules, to provide an enriched sample solution having a higher proportion of sample nucleic acid molecules comprising the target nucleic acid sequence than the sample solution. The instructions may further comprise repeating steps (b) and (c) one or more times on the enriched sample solution. The instructions may further comprise maintaining the temperature of the sample solution at or above a melting temperature of the plurality of sample nucleic acid molecules and the capture primer nucleic acid molecule.

The instructions for enzymatically extending the capture primer nucleic acid molecule may comprise: maintaining a temperature of the sample solution at or above a melting temperature of the plurality of sample nucleic acid molecules; introducing to the sample solution an extension enzyme configured to extend the capture primer nucleic acid molecule annealed to the target nucleic acid sequence; and maintaining the sample solution at about or below an annealing temperature of the capture primer nucleic acid molecule suitable to anneal the capture primer nucleic acid molecule to the target nucleic acid sequence; and maintaining the sample solution at about an extension temperature of the extension enzyme suitable for enzymatic extension by the extension enzyme of the capture primer nucleic acid molecule annealed to the target nucleic acid sequence.

The instructions for enzymatically degrading single-stranded sample nucleic acid molecules may comprise: introducing to the sample solution a degradation enzyme configured to degrade a single-stranded nucleic acid molecule comprising the universal adaptor nucleic acid sequence; and maintaining the temperature of the sample solution at a degradation temperature of the degradation enzyme.

The instructions may further comprise instructions for coupling universal adaptor molecules to samples nucleic acid molecules in a sample solution.

### DEPLETION KITS

The present disclosure provides a kit for depleting a sample nucleic acid molecule including a target nucleic acid sequence. The kit may comprise a capture primer nucleic acid molecule complementary to or partially complementary to a target sequence; and a degradation enzyme configured to degrade a double-stranded nucleic acid molecule.

As above, the kit includes a capture primer nucleic acid molecule.

The capture primer nucleic acid molecule may be perfectly complementary to a target nucleic acid sequence. The capture primer nucleic acid molecule may be partially complementary to one or more target nucleic acid molecules. As discussed further herein, the capture primer nucleic acid molecules can be at least partially complementary to a number of target nucleic acid sequences, and, thus, the kits of the present disclosure are configured to enrich sample nucleic acid molecules having a number of different target nucleic acid sequences, depending upon the reaction conditions in which they are deployed.

As discussed further herein with respect to FIGURE 2D, the capture primer nucleic acid molecules can be single stranded, at least partially double stranded, or double stranded, such as at an annealing temperature between the capture primer nucleic acid molecule and its target nucleic acid sequence.

The capture primer nucleic acid molecule may comprise a phosphorothioate linkage. In an instance, the phosphorothioate linkage is disposed between a base at a 3' end of the capture primer nucleic acid molecule and a base immediately adjacent to the base at the 3' end. Such phosphorothioate linkages are configured to resist 3' exonuclease activity, such as those present in proof reading polymerases.

The kit may further include a plurality of universal adaptor nucleic acid molecules configured to couple to a sample nucleic acid molecule. As discussed further herein with respect to the methods of the present disclosure, the universal adaptor nucleic acid molecules are suitable for use in a nucleic acid amplification reaction.

The universal adaptor nucleic acid molecule may include a nucleic acid sequence adjacent to a 3' end or a 5' end that is configured not to bind to itself, such as in a hairpin configuration, thus avoiding self-priming. The universal adaptor nucleic acid molecule may include a polyT sequence, a polyA sequence, or a combination thereof.

The kit may further comprise reagents for coupling the universal adaptor nucleic acid molecule to a sample nucleic acid molecule. The kit may comprise reagents from the group consisting of a transposase loaded with an oligonucleotide comprising a universal adaptor nucleic acid molecule; a restriction endonuclease, an oligonucleotide or oligonucleotide complex comprising a universal adaptor nucleic acid molecule, an oligonucleotide or oligonucleotide complex comprising a T7 promoter, an antibody or antibody fragment against a transcription factor, and combinations thereof.

The kits described herein include a degradation enzyme. In an instance, the degradation enzyme is configured to cleave double-stranded nucleic acid molecules comprising the universal adaptor nucleic acid molecule. In an instance, the degradation enzyme is configured to degrade double-stranded nucleic acid molecules comprising the universal adaptor nucleic acid molecule. The degradation enzyme may be a ribonuclease, or an endonuclease. The endonuclease may be an endoribonuclease. In an instance, the degradation enzyme is not a restriction endonuclease.
The degradation enzyme may be a ribonuclease, or an endonuclease. The endonuclease may be an endoribonuclease, and may be selected from the group consisting of Rnase HII, RNase H, Rnase III, and combinations thereof.

In an instance, the degradation enzyme is Rnase HII. In an instance, the degradation enzyme is according to SEQ ID NO. 16. In an instance, the degradation has a sequence homology to SEQ ID NO. 16 greater to 90%, greater than 95%, or greater than 99%.

In an instance, the degradation enzyme is Rnase H. In an instance, the degradation enzyme is according to SEQ ID NO. 17. In an instance, the degradation has a sequence homology to SEQ ID NO. 17 greater to 90%, greater than 95%, or greater than 99%.

In an instance, the degradation enzyme is Rnase III. In an instance, the degradation enzyme is according to SEQ ID NO. 18. In an instance, the degradation has a sequence homology to SEQ ID NO. 18 greater to 90%, greater than 95%, or greater than 99%.

The kit may further comprise an extension enzyme configured to extend a capture primer nucleic acid molecule annealed to the target nucleic acid sequence.

The extension enzyme may be selected from the group consisting of a polymerase, a reverse transcriptase, and combinations thereof.

The kit may further comprise instructions for depleting a target nucleic acid sequence, such as in a sample comprising sample nucleic acid molecules.

The instructions may comprise instructions for performing the depletion methods of the present disclosure. The instructions may comprise (a) introducing to a sample solution, comprising a plurality of sample nucleic acid molecules each comprising a universal adaptor nucleic acid sequence comprising ribonucleotides, a capture primer nucleic acid molecule complementary or partially complementary to a target nucleic acid sequence of one or more sample nucleic acid molecules of the plurality of sample nucleic acid molecules; (b) enzymatically extending the capture primer nucleic acid molecule annealed to the target nucleic acid sequence of the one or more sample nucleic acid molecules; and (c) enzymatically cleaving double-stranded ribonucleic acid molecules of the sample nucleic acid molecules, to provide a depleted sample solution having a lower proportion of sample nucleic acid molecules comprising the target nucleic acid sequence than the sample solution. The instructions may further comprise repeating steps (b) and (c) one or more times on the enriched sample solution.

The instructions may further comprise maintaining the temperature of the sample solution at or above a melting temperature of the plurality of sample nucleic acid molecules and the capture primer nucleic acid molecule.

The instructions for enzymatically extending the capture primer nucleic acid molecule may comprise maintaining a temperature of the sample solution at or above a melting temperature of the plurality of sample nucleic acid molecules; introducing to the sample solution an extension enzyme configured to extend the capture primer nucleic acid molecule annealed to the target nucleic acid sequence; and maintaining the sample solution at about or below an annealing temperature of the capture primer nucleic acid molecule suitable to anneal the capture primer nucleic acid molecule to the target nucleic acid sequence; and maintaining the sample solution at about an extension temperature of the extension enzyme suitable for enzymatic extension by the extension enzyme of the capture primer nucleic acid molecule annealed to the target nucleic acid sequence.

The instructions for enzymatically cleaving double-stranded sample nucleic acid molecules may comprise introducing to the sample solution a degradation enzyme configured to cleave a double-stranded nucleic acid molecule comprising the universal adaptor nucleic acid sequence; and maintaining the temperature of the sample solution at a degradation temperature of the degradation enzyme.

The instructions may further comprise: introducing a plurality of amplification primer nucleic acid molecules to the depleted sample solution, wherein amplification primer nucleic acid molecules of the plurality of amplification primer nucleic acid molecules are complementary to the universal adaptor nucleic acid sequence; and performing a nucleic acid amplification reaction on the plurality of sample nucleic acid molecules in the depleted sample solution with the plurality of amplification primer nucleic acid molecules.

The instructions may further comprise instructions for coupling universal adaptor molecules to samples nucleic acid molecules in a sample solution.

### EXAMPLES

### EXAMPLE 1: EXAMPLE RESULTS OF ENRICHMENT STRATEGY USING SELECTION PROBES:

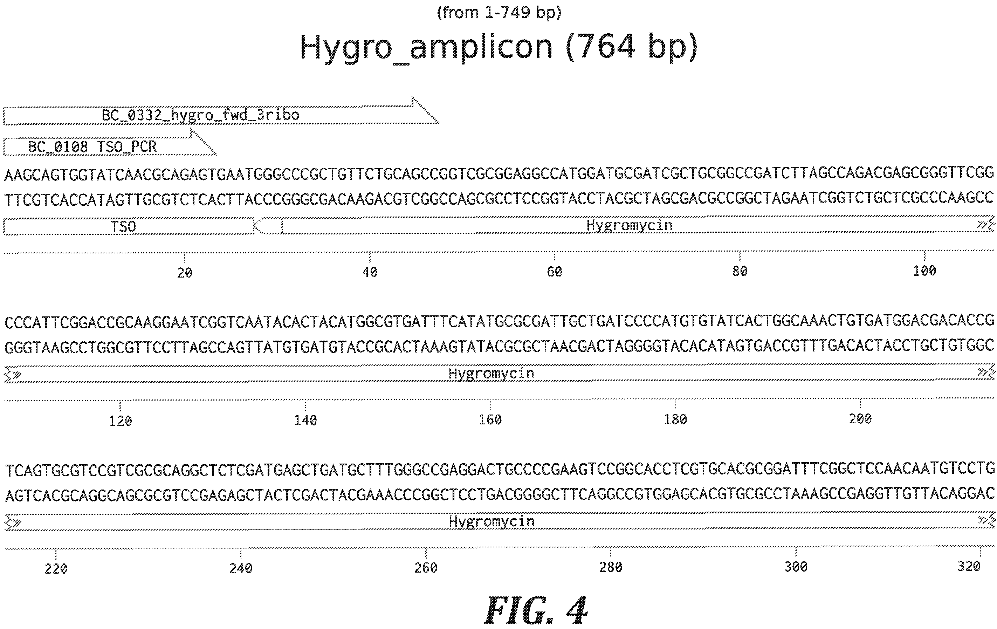

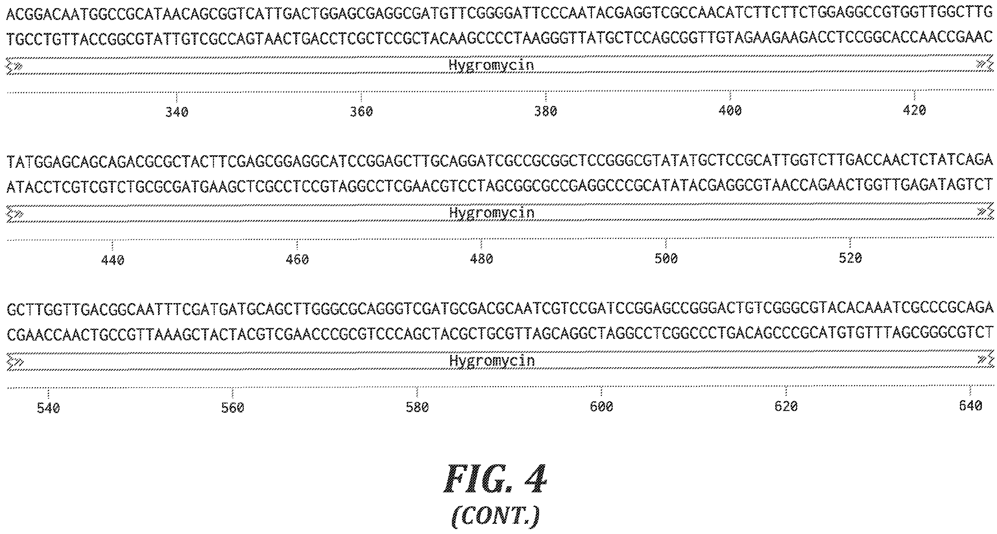

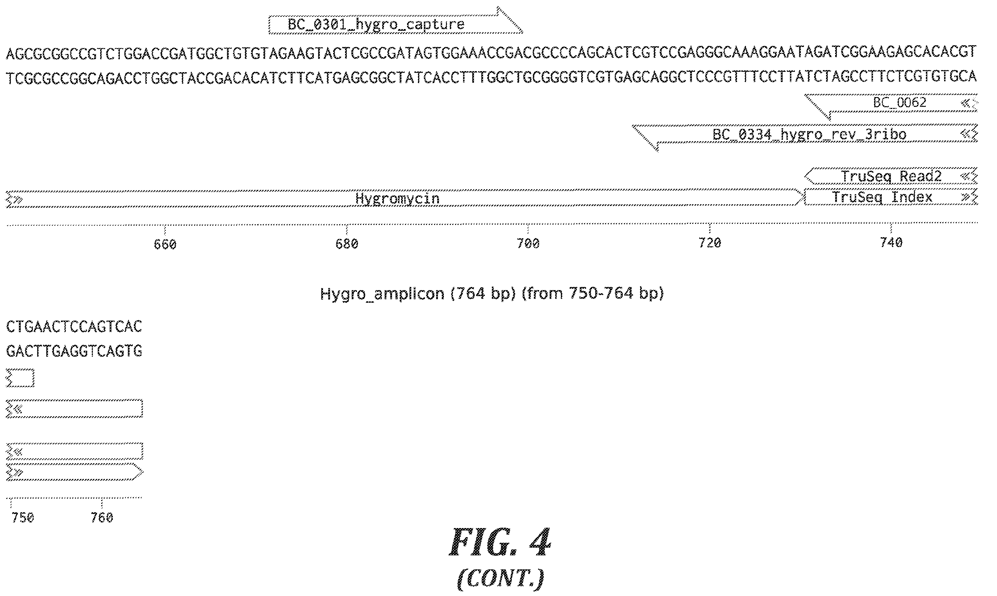

Two different amplicons of different length with universal adapters were generated by amplifying sequences from a plasmid (AmpR: 421 bp and Hygro: 774 bp). Primers BC_0328 and BC_0330 were used to generate the AmpR amplicon (Figure 6). Primers BC_0332 and BC_0334 were used to generate the Hygro amplicon (Figure 4).

Equal amounts of both amplicons (0.2ng each) were added to 20uL reactions.

To enrich for the AmpR amplicon, we used the following mix, where BC_306_amp_capture is an oligonucleotide that is complementary to the AmpR amplicon, but not the Hygro amplicon.

**TABLE 1:**

| Component | 20 uL reaction |
|---|---|
| 10x Standard Taq buf | 2 |
| 10mM DNTPs | 0.4 |
| Amp + Hygro amplicon mix (0.4ng total) | 1 |
| Taq DNA Polymerase | 0.1 |
| BC_0306_amp_capture | 0.4 |
| 10x Diluted RNase T1 | 1 |
| Nuclease-free H2O | 15.1 |
| Total Volume | 20 |

To enrich for the Hygro amplicon, we used the following mix, where BC_301_hygro_capture is an oligonucleotide that is complementary to the Hygro amplicon, but not the AmpR amplicon.

**TABLE 2:**

| Component | 20 uL reaction |
|---|---|
| 10x Standard Taq buf | 2 |
| 10mM DNTPs | 0.4 |
| Amp + Hygro amplicon mix (0.4ng total) | 1 |
| Taq DNA Polymerase | 0.1 |
| BC_0301_hygro_capture | 0.4 |
| 10x Diluted RNase T1 | 1 |
| Nuclease-free H2O | 15.1 |
| Total Volume | 20 |

Samples were then cycled with the following conditions:
Thermocycle samples with the following protocol for 1 or 3 cycles:
1. 95°C - 30s
2. 58°C - 20s
3. 68°C - 20s
4. 37°C or 42C or 50C - 15 min

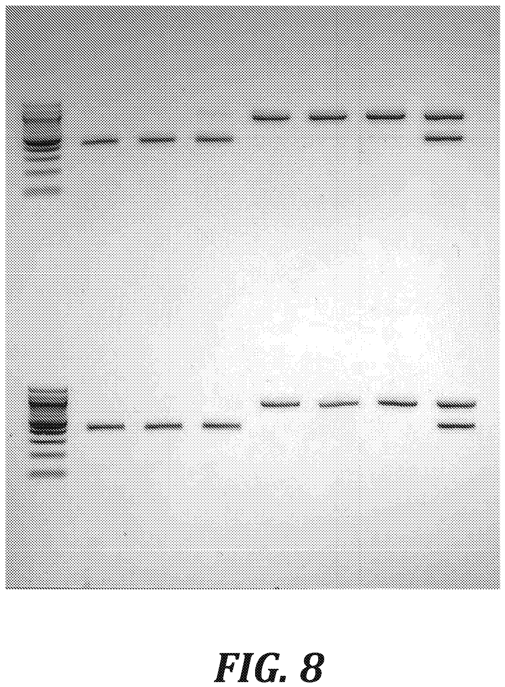

Then samples were immediately put on ice
2 uL of each reaction was then added to a 25 uL qPCR reaction with universal primers. Once reactions began to plateau, they were removed from qPCR and run on a 1.25%, agarose gel. The results are shown in FIGURE 8.

### Left to right in top row:

1. 100 base-pair ladder (New England Biolabs)
2. Amp capture, 1 cycle, step 4 at 37°C
3. Amp capture, 1 cycle, step 4 at 42°C
4. Amp capture, 1 cycle, step 4 at 50°C
5. Hygro capture, 1 cycle, step 4 at 37°C
6. Hygro capture, 1 cycle, step 4 at 42°C
7. Hygro capture, 1 cycle, step 4 at 50°C
8. Control: DNA only

### Left to right in bottom row:

9. 100 base-pair ladder (New England Biolabs)
10. Amp capture, 3 cycles, step 4 at 37°C
11. Amp capture, 3 cycles, step 4 at 42°C
12. Amp capture, 3 cycles, step 4 at 50°C
13. Hygro capture, 3 cycles, step 4 at 37°C
14. Hygro capture, 3 cycles, step 4 at 42°C
15. Hygro capture, 3 cycles, step 4 at 50°C
16. Control: DNA only

### Oligonucleotide sequences:

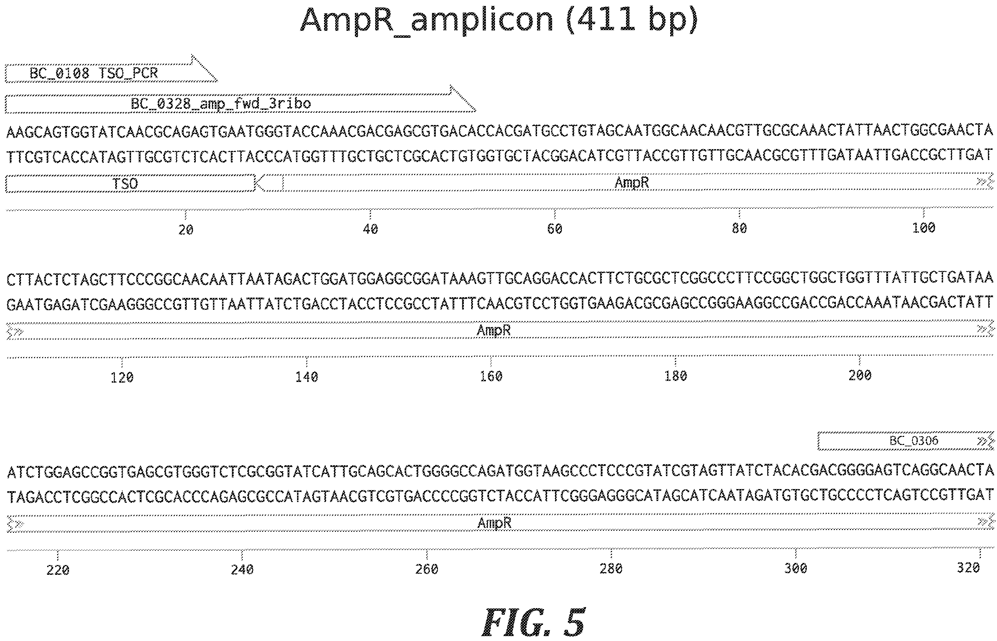

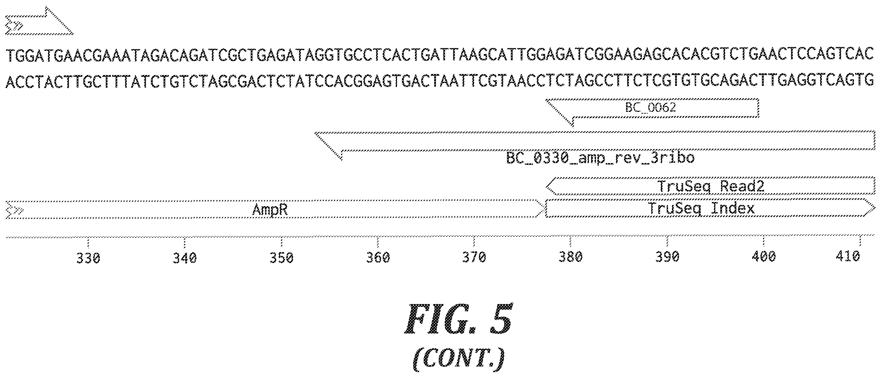

See Figure 5: AmpR_amplicon-sequence.pdf
See Figure 4: Hygro_amplicon-sequence.pdf
BC_0108_TSO_PCR AAGCAGTGGTATCAACGCAGAGT (SEQ ID NO. 12)
BC_0062_Primer_Bind CAGACGTGTGCTCTTCCGATCT (SEQ ID NO. 13)
BC_0328_amp_fwd_3ribo 
AAGCAGTGGTATCAACrGCArGAGTrGAATGGGTACCAAACGACGAGCG
TGACA (SEQ ID NO. 1)
BC_0330_amp_rev_3ribo 
GTGACTGGAGTTCAGACrGTGTrGCTCTTCCrGATCTCCAATGCTTAATC
AGTGAGGCACC (SEQ ID NO. 2)
BC_0306_Amp_capture ACGGGGAGTCAGGCAACTATGGATGA (SEQ ID NO. 19)
BC_0359_amp_ribo_dT_fwd 
TTTTTTTTTTAAGCAGTGGTATCAACrGCArGAGTrGAATGGGTACCAAA
CGACGAGCGTGACA (SEQ ID NO. 20)
BC_0360_amp_ribo_dT_rev 
TTTTTTTTTTCAGACrGTGTrGCTCTTCCrGATCTCCAATGCTTAATCAGT
GAGGCACC (SEQ ID NO. 21)
BC_0332_hygro_fwd_3ribo 
AAGCAGTGGTATCAACrGCArGAGTrGAATGGGCCCGCTGTTCTGCAGC
C (SEQ ID NO. 3)
BC_0334_hygro_rev_3ribo 
GTGACTGGAGTTCAGACrGTGTrGCTCTTCCrGATCTATTCCTTTGCCCTC
GGACG (SEQ ID NO. 4)
BC_0301_hygro_capture AGAAGTACTCGCCGATAGTGGAAACCGA (SEQ ID NO. 22)

The results from the gel image in Figure 8 show enrichment of the desired target molecules across a variety of conditions. Lanes 2-4 and 10-12 show enrichment of the AmpR molecules. Lanes 5-7 and 13-15 show enrichment of the Hygro molecules. Enrichment occurs across a range of temperatures for the degradation step (37°C -50°C). The gel also shows that multiple cycles of melting nucleic acids, annealing capture primers, extending capture primers, and degrading single stranded riboguanines can lead to equivalent or higher fold enrichment than a single cycle (compare lanes 10-12 to lanes 4-6 and lanes 13-15 to lanes 5-7).

### EXAMPLE 2:

In this example, single-cell RNA-sequencing libraries (from expanded primary T-cells) were enriched for specific sequences matching parts of the following genes: ACTB (ATGGCCCAGTCCTCTCCCAA, SEQ ID NO. 5), GAPDH (AGGAGTAAGACCCCTGGACCAC, SEQ ID NO. 6), TRAC (AGAACCCTGACCCTGCCG, SEQ ID NO. 7), TRBC1 (CTGAAAAACGTGTTCCCACCCGAG, SEQ ID NO. 8), and TRBC2 (ACCTGAACAAGGTGTTCCCACC, SEQ IDNO. 9)).

TRAC corresponds to the constant region of the T cell receptor alpha chain, while TRBC1 and TRCB2 correspond to two possible constant regions of the T cell receptor beta chain. T cell receptor alpha and beta chains are generated by VJ and VDJ recombination leading to a very high diversity of possible sequences for each. However, by enriching nucleic acid sequences containing part of the TRAC sequence, it is possible to enrich all or nearly all nucleic acid sequences coding for the T cell receptor alpha chain, and similarly by enriching nucleic acid sequences containing part of either the TRBC1 or TRBC2, it is possible to enrich all or nearly all nucleic acid sequences coding for the T cell receptor beta chain.

A single-cell RNA-sequencing library of amplified cDNA was generated according the published SPLiT-seq method. 1 ng of amplified cDNA was reamplified for 11 cycles of PCR using primers BC_385 and BC_386 to introduce riboguanosines into to each 5' end of the double stranded DNA molecules. The resulting PCR products were purified with SPRI beads (Kapa Pure Beads) using a 2:1 ratio of beads to PCR product according to the manufacturer's instructions. The concentration of the resulting purified PCR product was measured using the Qubit dsDNA HS Assay Kit.

In total 12 different variations of enrichment were compared. 3 different polymerase mixes were tested, two different polymerase extension times were tested, and two concentrations of Rnase T1 were tested (3 x 2 x 2 = 12 combinatorial variations).
Variation 1 (Hot start Taq in 1x Standard Taq Buffer, 30 s polymerase extension, 100u Rnase T1)
Variation 2 (Hot start Taq in 1x Standard Taq Buffer, 120 s polymerase extension, 100u Rnase T1)
Variation 3 (Hot start Taq in 1x Standard Taq Buffer, 30 s polymerase extension, 20u Rnase T1)
Variation 4 (Hot start Taq in 1x Standard Taq Buffer, 120 s polymerase extension, 20u Rnase T1)
Variation 5 (OneTaq Hot start in 1x OneTaq Standard Reaction Buffer, 30 s polymerase extension, 100u Rnase T1)
Variation 6 (OneTaq Hot start in 1x OneTaq Standard Reaction Buffer, 120 s polymerase extension, 100u Rnase T1)
Variation 7 (OneTaq Hot start in 1x OneTaq Standard Reaction Buffer, 30 s polymerase extension, 20u Rnase T1)
Variation 8 (OneTaq Hot start in 1x OneTaq Standard Reaction Buffer, 120 s polymerase extension, 20u Rnase T1)
Variation 9 (Deep Vent Exo- in 1x ThermoPol Reaction Buffer, 30 s polymerase extension, 100u Rnase T1)
Variation 10 (Deep Vent Exo- in 1x ThermoPol Reaction Buffer, 120 s polymerase extension, 100u Rnase T1)
Variation 11 (Deep Vent Exo- in 1x ThermoPol Reaction Buffer, 30 s polymerase extension, 20u Rnase T1)
Variation 12 (Deep Vent Exo- in 1x ThermoPol Reaction Buffer, 120 s polymerase extension, 20u Rnase T1)

Each reaction was prepared with:
(2 uL 10x Standard Taq Buffer/4uL OneTaq Standard Reaction Buffer/2 uL ThermoPol Reaction Buffer), 1.6uL 2.5mM dNTPs, (0.1uL HotStart Taq Polymerase/0.1 uL OneTaq^{®} Hot Start DNA Polymerase/0.1uL Deep Vent^{®} (exo-) DNA Polymerase), 1 uL of pooled capture primers (10 uM total, 2 uM each), (11.3/13.3uL water), 1 uL of amplified cDNA (from PCR using BC_385 and BC_386), and 1uL of Rnase T1 (diluted to 100u/uL or 20u/uL). Primers BC_0344_ACTB_probe (SEQ ID NO. 5), BC_0343_GAPDH_probe (SEQ ID NO. 6), BC_0391_TRAC_probe (SEQ ID NO. 7), BC_0392_TRBC1_probe (SEQ ID NO. 8), BC_0393_TRBC2_probe (SEQ ID NO. 9) were used as the pooled capture primers.

Variations 1, 3, 5, 7 were cycled as follows:
a. 95C for 30s, b. 95C for 30s, c. 53C for 20s, d. 68C for 30s, e. 37C for 15 min, f. repeat steps b-e 2 additional cycles (3 including first cycle).

Variations 2, 4, 6, 8 were cycled as follows:
a. 95C for 30s, b. 95C for 30s, c. 53C for 20s, d. 68C for 2min, e. 37C for 15 min, f. repeat steps b-e 2 additional cycles (3 including first cycle).

Variations 9 and 11 were cycled as follows:
a. 95C for 30s, b. 95C for 30s, c. 55C for 20s, d. 72C for 30s, e. 37C for 15 min, f. repeat steps b-e 2 additional cycles (3 including first cycle).

Variations 10 and 12 were cycled as follows:
a. 95C for 30s, b. 95C for 30s, c. 55C for 20s, d. 72C for 2min, e. 37C for 15 min, f. repeat steps b-e 2 additional cycles (3 including first cycle).

All 12 reactions were then purified using a single sided SPRI cleanup (Kapa Pure Beads) according to the manufacturer's instructions (2x ratio of beads to PCR product). Each of the 12 purified reactions were then amplified with PCR using primers BC_0062 (SEQ ID NO. 12) and BC_0108 TSO_PCR (SEQ ID NO. 12). The amplified PCR products were then prepared for next generation sequencing on an Illumina sequencer by fragmentation, end-repair (including A-tailing), adapter ligation, and PCR with primers to add indexed Illumina adapters (P7 and P5).

The original amplified cDNA library (which did not undergo any enrichment) was also prepared for next generation sequencing using the same methods (fragmentation, end-repair (including A-tailing), adapter ligation, and PCR with primers to add indexed Illumina adapters (P7 and P5)).

All 13 libraries (12 variations of enrichments and original non-enriched library) were sequenced together on an Illumina NextSeq. The resulting libraries were demultiplexed according to indices added during the final PCR.

The fold-change enrichment for each of the 12 enrichment variations relative to the non-enriched library was then calculated for each of the 5 sequences that were intended to be enriched: ACTB (ATGGCCCAGTCCTCTCCCAA, SEQ ID NO. 5), GAPDH (AGGAGTAAGACCCCTGGACCAC, SEQ ID NO.6),TRAC (AGAACCCTGACCCTGCCG, SEQ ID NO.7),TRBC1 (CTGAAAAACGTGTTCCCACCCGAG, SEQ ID NO. 8), and TRBC2 (ACCTGAACAAGGTGTTCCCACC, SEQ ID NO. 9)).

**TABLE 3: Fold-change Enrichment:**

| Variation | Rnase T1 | Polymerase | Poly_ext_time | ACTB | GAPDH | TRAC | TRBC1 | TRBC2 |
|---|---|---|---|---|---|---|---|---|
| **1** | 100U | Hocstart_Taq | 30s | 1.61 | 1.35 | 3.31 | 2.12 | 1.85 |
| **2** | 100U | Hotstart_Taq | 120s | 2.61 | 2.01 | 4.41 | 2.81 | 2.58 |
| **3** | 20U | Hotstart_Taq | 30s | 7.75 | 6.91 | 10.59 | 8.04 | 8.09 |
| **4** | 20U | Hotstart_Taq | 120s | 12.77 | 12.50 | 15.76 | 15.03 | 15.78 |
| **5** | 100U | OneTaq_hotstart | 30s | 4.35 | 4.12 | 10.23 | 8.58 | 7.74 |
| **6** | 100U | OneTaq_hotstart | 120s | 5.03 | 5.26 | 10.86 | 9.87 | 8.97 |
| **7** | 20U | OneTaq_hotstart | 30s | 6.51 | 7.49 | 9.49 | 9.11 | 9.08 |
| **8** | 20U | OneTaq_hotstart | 120s | 8.88 | 11.31 | 12.44 | 13.48 | 13.26 |
| **9** | 100U | Deep_Vent_exo | 30s | 14.90 | 11.38 | 20.42 | 16.56 | 17.01 |
| **10** | 100U | Deep_Vent_exo | 120s | 16.10 | 18.19 | 20.39 | 20.35 | 20.87 |
| **11** | 20U | Deep_Vent_exo | 30s | 13.67 | 8.89 | 16.80 | 13.99 | 14.31 |
| **12** | 20U | Deep_Vent_exo | 120s | 14.36 | 14.77 | 17.33 | 18.28 | 18.07 |

The results in Table 3 show enrichment of the desired target molecules across a variety of conditions. For each of the five target sequences, nucleic acids containing the given sequence are enriched across different experimental conditions. The concentration of Rnase T1, type of polymerase, and polymerase extension time can be adjusted resulting in different fold enrichment of the target sequences.

## Claims

1. A method for enriching a target nucleic acid sequence, the method comprising:
(a) introducing to a sample solution, comprising a plurality of sample nucleic acid molecules each comprising a universal adaptor nucleic acid sequence, a capture primer nucleic acid molecule complementary to or partially complementary to a target nucleic acid sequence of one or more sample nucleic acid molecules of the plurality of sample nucleic acid molecules;
(b) enzymatically extending the capture primer nucleic acid molecule annealed to the target nucleic acid sequence of the one or more sample nucleic acid molecules; and
(c) enzymatically degrading single-stranded sample nucleic acid molecules, to provide an enriched sample solution having a higher proportion of sample nucleic acid molecules comprising the target nucleic acid sequence than the sample solution.

2. The method of claim 1, wherein enzymatically extending the capture primer nucleic acid molecule comprises:
maintaining a temperature of the sample solution at or above a melting temperature of the plurality of sample nucleic acid molecules;
introducing to the sample solution an extension enzyme configured to extend the capture primer nucleic acid molecule annealed to the target nucleic acid sequence;
maintaining the sample solution at about or below an annealing temperature of the capture primer nucleic acid molecule suitable to anneal the capture primer nucleic acid molecule to the target nucleic acid sequence; and
maintaining the sample solution at about an extension temperature of the extension enzyme suitable for enzymatic extension by the extension enzyme of the capture primer nucleic acid molecule annealed to the target nucleic acid sequence.

3. The method of claim 2, wherein the extension enzyme is selected from the group consisting of a polymerase, a reverse transcriptase, and combinations thereof.

4. The method of any of claims 1-3, wherein enzymatically degrading single-stranded sample nucleic acid molecules comprises:
introducing to the sample solution a degradation enzyme configured to degrade a single-stranded nucleic acid molecule comprising the universal adaptor nucleic acid sequence; and
maintaining the temperature of the sample solution at a degradation temperature of the degradation enzyme.

5. The method of claim 4, wherein the degradation enzyme is a ribonuclease.

6. The method of claim 4 or 5, wherein the degradation enzyme is an endonuclease.

7. The method of claim 6, wherein the endonuclease is an endoribonuclease.

8. The method of claim 7, wherein the endoribonuclease is selected from the group consisting of Rnase T1, Rnase A, and combinations thereof.

9. The method of any of claims 4-8, wherein the degradation enzyme is Rnase T1, and wherein the universal adaptor nucleic acid sequence comprises a riboguanine.

10. The method of any of claim 4-8, wherein the degradation enzyme is Rnase A, and wherein the universal adaptor nucleic acid sequence comprises base selected from the group consisting of a ribocytosine, a ribouracil, and combinations thereof.

11. The method of any of claims 1-10, wherein enzymatically degrading the single-stranded sample nucleic acid molecules includes degrading a portion of the universal adaptor nucleic acid sequence disposed on the single-stranded sample nucleic acid molecules.

12. The method of any of claims 2-11, further comprising repeating steps (b) and (c) one or more times on the enriched sample solution.

13. The method of any one of claims 1-12, further comprising:
introducing a plurality of amplification primer nucleic acid molecules to the enriched sample solution, wherein amplification primer nucleic acid molecules of the plurality of amplification primer nucleic acid molecules are complementary to the universal adaptor nucleic acid sequence; and
performing a nucleic acid amplification reaction on the plurality of sample nucleic acid molecules in the enriched sample solution with the plurality of amplification primer nucleic acid molecules to provide an amplified enriched sample solution.

14. The method of claim 13, wherein performing the nucleic acid amplification reaction on the plurality of sample nucleic acid molecules in the enriched sample solution does not or does not substantially amplify sample nucleic acid molecules that have been degraded by the degradation enzyme.

15. The method of any of claims 1-14, further comprising sequencing nucleic acid molecules in the enriched sample solution.

## Patentansprüche

1. Ein Verfahren zur Anreicherung einer Zielnukleinsäuresequenz, wobei das Verfahren Folgendes umfasst:
(a) Einführen, in eine Probenlösung, die eine Vielzahl von Probennukleinsäuremolekülen umfasst, die jeweils eine universelle Adapternukleinsäuresequenz umfassen, eines Fangprimer-Nukleinsäuremoleküls, das komplementär zu oder teilweise komplementär zu einer Zielnukleinsäuresequenz von einem oder mehreren Probennukleinsäuremolekülen der Vielzahl von Probennukleinsäuremolekülen ist;
(b) enzymatisches Verlängern des Fangprimer-Nukleinsäuremoleküls, das an die Zielnukleinsäuresequenz des einen oder der mehreren Probennukleinsäuremoleküle angelagert wird; und
(c) enzymatisches Abbauen einzelsträngiger Probennukleinsäuremoleküle, um eine angereicherte Probenlösung bereitzustellen, die einen höheren Anteil von die Zielnukleinsäuresequenz umfassenden Probennukleinsäuremolekülen aufweist als die Probenlösung.

2. Verfahren nach Anspruch 1, wobei das enzymatische Verlängern des Fangprimer-Nukleinsäuremoleküls Folgendes umfasst:
Halten einer Temperatur der Probenlösung bei oder über einer Schmelztemperatur der Vielzahl von Probennukleinsäuremolekülen;
Einführen eines Verlängerungsenzyms in die Probenlösung, das dazu ausgelegt ist, das an die Zielnukleinsäuresequenz angelagerte Fangprimer-Nukleinsäuremolekül zu verlängern;
Halten der Probenlösung bei etwa oder unter einer Anlagerungstemperatur des Fangprimer-Nukleinsäuremoleküls, die zum Anlagern des Fangprimer-Nukleinsäuremoleküls an die Zielnukleinsäuresequenz geeignet ist; und
Halten der Probenlösung bei etwa einer Verlängerungstemperatur des Verlängerungsenzyms, die zur enzymatischen Verlängerung durch das Verlängerungsenzym des an die Zielnukleinsäuresequenz angelagerten Fangprimer-Nukleinsäuremoleküls geeignet ist.

3. Verfahren nach Anspruch 2, wobei das Verlängerungsenzym aus der Gruppe ausgewählt ist, die aus einer Polymerase, einer reversen Transkriptase und Kombinationen davon besteht.

4. Verfahren nach einem der Ansprüche 1-3, wobei das enzymatische Abbauen von einzelsträngigen Probennukleinsäuremolekülen Folgendes umfasst:
Einführen, in die Probenlösung, eines Abbauenzyms, das dazu ausgelegt ist, ein einzelsträngiges Nukleinsäuremolekül abzubauen, das die universelle Adapternukleinsäuresequenz umfasst; und
Halten der Temperatur der Probenlösung bei einer Abbautemperatur des Abbauenzyms.

5. Verfahren nach Anspruch 4, wobei das Abbauenzym eine Ribonuklease ist.

6. Verfahren nach Anspruch 4 oder 5, wobei das Abbauenzym eine Endonuklease ist.

7. Verfahren nach Anspruch 6, wobei die Endonuklease eine Endoribonuklease ist.

8. Verfahren nach Anspruch 7, wobei die Endoribonuklease aus der Gruppe ausgewählt ist, die aus Rnase T1, Rnase A und Kombinationen davon besteht.

9. Verfahren nach einem der Ansprüche 4-8, wobei das Abbauenzym Rnase T1 ist und wobei die universelle Adapternukleinsäuresequenz ein Riboguanin umfasst.

10. Verfahren nach einem der Ansprüche 4-8, wobei das Abbauenzym Rnase A ist und wobei die universelle Adapternukleinsäuresequenz eine Base umfasst, die aus der Gruppe ausgewählt ist, die aus einem Ribocytosin, einem Ribouracil und Kombinationen davon besteht.

11. Verfahren nach einem der Ansprüche 1-10, wobei das enzymatische Abbauen der einzelsträngigen Probennukleinsäuremoleküle das Abbauen eines Anteils der universellen Adapternukleinsäuresequenz, der auf den einzelsträngigen Probennukleinsäuremolekülen angeordnet ist, einschließt.

12. Verfahren nach einem der Ansprüche 2-11, das ferner das einmalige oder mehrmalige Wiederholen der Schritte (b) und (c) an der angereicherten Probenlösung umfasst.

13. Verfahren nach einem der Ansprüche 1-12, das ferner Folgendes umfasst:
Einführen einer Vielzahl von Amplifikationsprimer-Nukleinsäuremolekülen in die angereicherte Probenlösung, wobei die Amplifikationsprimer-Nukleinsäuremoleküle der Vielzahl von Amplifikationsprimer-Nukleinsäuremolekülen komplementär zu der universellen Adapternukleinsäuresequenz sind; und
Durchführen einer Nukleinsäureamplifikationsreaktion an der Vielzahl von Probennukleinsäuremolekülen in der angereicherten Probenlösung mit der Vielzahl von Amplifikationsprimer-Nukleinsäuremolekülen, um eine amplifizierte angereicherte Probenlösung bereitzustellen.

14. Verfahren nach Anspruch 13, wobei das Durchführen der Nukleinsäureamplifikationsreaktion an der Vielzahl von Probennukleinsäuremolekülen in der angereicherten Probenlösung die durch das Abbauenzym abgebauten Probennukleinsäuremoleküle nicht oder nicht wesentlich amplifiziert.

15. Verfahren nach einem der Ansprüche 1-14, das ferner das Sequenzieren von Nukleinsäuremolekülen in der angereicherten Probenlösung umfasst.

## Revendications

1. Procédé d'enrichissement d'une séquence d'acide nucléique cible, le procédé comprenant :
(a) l'introduction dans une solution d'échantillon, comprenant une pluralité de molécules d'acide nucléique échantillon comprenant chacune une séquence d'acide nucléique adaptatrice universelle, d'une molécule d'acide nucléique qui est une amorce de capture complémentaire ou partiellement complémentaire d'une séquence d'acide nucléique cible d'une ou plusieurs molécules d'acide nucléique échantillon de la pluralité de molécules d'acide nucléique échantillon ;
(b) l'extension par voie enzymatique de la molécule d'acide nucléique qui est une amorce de capture hybridée avec la séquence d'acide nucléique cible des une ou plusieurs molécules d'acide nucléique échantillon ; et
(c) la dégradation par voie enzymatique des molécules d'acide nucléique échantillon monobrin, pour produire une solution d'échantillon enrichie contenant une plus grande proportion de molécules d'acide nucléique échantillon comprenant la séquence d'acide nucléique cible que la solution d'échantillon.

2. Procédé selon la revendication 1, dans lequel l'extension par voie enzymatique de la molécule d'acide nucléique qui est une amorce de capture comprend :
le maintien d'une température de la solution d'échantillon à une température égale ou supérieure à une température de fusion de la pluralité de molécules d'acide nucléique échantillon ;
l'introduction dans la solution d'échantillon d'une enzyme d'extension configurée pour étendre la molécule d'acide nucléique qui est une amorce de capture hybridée avec la séquence d'acide nucléique cible ;
le maintien de la solution d'échantillon à une température environ égale ou inférieure à une température d'hybridation de la molécule d'acide nucléique qui est une amorce de capture convenant pour l'hybridation de la molécule d'acide nucléique qui est une amorce de capture avec la séquence d'acide nucléique cible ; et
le maintien de la solution d'échantillon à une température environ égale à une température d'extension de l'enzyme d'extension convenant pour l'extension par voie enzymatique par l'enzyme d'extension de la molécule d'acide nucléique qui est une amorce de capture hybridée avec la séquence d'acide nucléique cible.

3. Procédé selon la revendication 2, dans lequel l'enzyme d'extension est sélectionnée dans le groupe constitué d'une polymérase, d'une transcriptase inverse, et de combinaisons de celles-ci.

4. Procédé selon l'une quelconque des revendications 1 à 3, dans lequel la dégradation par voie enzymatique des molécules d'acide nucléique échantillon monobrin comprend :
l'introduction dans la solution d'échantillon d'une enzyme de dégradation configurée pour dégrader une molécule d'acide nucléique monobrin comprenant la séquence d'acide nucléique adaptatrice universelle ; et
le maintien de la température de la solution d'échantillon à une température de dégradation de l'enzyme de dégradation.

5. Procédé selon la revendication 4, dans lequel l'enzyme de dégradation est une ribonucléase.

6. Procédé selon la revendication 4 ou 5, dans lequel l'enzyme de dégradation est une endonucléase.

7. Procédé selon la revendication 6, dans lequel l'endonucléase est une endoribonucléase.

8. Procédé selon la revendication 7, dans lequel l'endoribonucléase est sélectionnée dans le groupe constitué de la Rnase T1, de la Rnase A, et de combinaisons de celles-ci.

9. Procédé selon l'une quelconque des revendications 4 à 8, dans lequel l'enzyme de dégradation est la Rnase T1, et dans lequel la séquence d'acide nucléique adaptatrice universelle comprend une riboguanine.

10. Procédé selon l'une quelconque des revendications 4 à 8, dans lequel l'enzyme de dégradation est la Rnase A, et dans lequel la séquence d'acide nucléique adaptatrice universelle comprend une base sélectionnée dans le groupe constitué d'une ribocytosine, d'un ribouracile, et de combinaisons de ceux-ci.

11. Procédé selon l'une quelconque des revendications 1 à 10, dans lequel la dégradation par voie enzymatique des molécules d'acide nucléique échantillon monobrin comprend la dégradation d'une partie de la séquence d'acide nucléique adaptatrice universelle disposée sur les molécules d'acide nucléique échantillon monobrin.

12. Procédé selon l'une quelconque des revendications 2 à 11, comprenant en outre une répétition des étapes (b) et (c) une ou plusieurs fois sur la solution d'échantillon enrichie.

13. Procédé selon l'une quelconque des revendications 1 à 12, comprenant en outre :
l'introduction d'une pluralité de molécules d'acide nucléique qui sont des amorces d'amplification dans la solution d'échantillon enrichie, les molécules d'acide nucléique qui sont des amorces d'amplification de la pluralité de molécules d'acide nucléique qui sont des amorces d'amplification étant complémentaires de la séquence d'acide nucléique adaptatrice universelle ; et
la réalisation d'une réaction d'amplification d'acide nucléique sur la pluralité de molécules d'acide nucléique échantillon dans la solution d'échantillon enrichie avec la pluralité de molécules d'acide nucléique qui sont des amorces d'amplification pour produire une solution d'échantillon enrichie amplifiée.

14. Procédé selon la revendication 13, dans lequel la réalisation de la réaction d'amplification d'acide nucléique sur la pluralité de molécules d'acide nucléique échantillon dans la solution d'échantillon enrichie n'amplifie pas ou n'amplifie pas sensiblement les molécules d'acide nucléique échantillon qui ont été dégradées par l'enzyme de dégradation.

15. Procédé selon l'une quelconque des revendications 1 à 14, comprenant en outre le séquençage de molécules d'acide nucléique dans la solution d'échantillon enrichie.
